# EUROPEAN PATENT SPECIFICATION

(11) **EP 4 077 720 B1**
(45) Date of publication and mention of the grant of the patent: **09.10.2024**
(21) Application number: 20901413.3
(22) Date of filing: 18.12.2020
(51) Int. Cl.: C12Q 1/6886

(54) **MULTIPLEXED ASSAY USING DIFFERENTIAL FRAGMENT SIZE TO IDENTIFY CANCER SPECIFIC CELL-FREE DNA**
MULTIPLEXTEST UNTER VERWENDUNG VON DIFFERENZIELLER FRAGMENTGRÖSSE ZUR IDENTIFIZIERUNG VON KREBSSPEZIFISCHER ZELLFREIER DNA
ANALYSE MULTIPLEXE UTILISANT UNE TAILLE DE FRAGMENT DIFFÉRENTIELLE POUR IDENTIFIER DE L'ADN ACELLULAIRE SPÉCIFIQUE AU CANCER

(30) Priority: 19.12.2019 US 201962950830 P
(43) Date of publication of application: 26.10.2022
(73) Proprietor: Cadex Genomics, Corp., St. Louis, MO 63110 (US)
(72) Inventor: SINHA, Sudhir, Metairie, Louisiana 70006 (US); SPITZER, Gary, Greenville, South Carolina 29605 (US); BROWN, Hiromi, Baton Rouge, Louisiana 70810 (US); HALL, Patrick, Edmond, Oklahoma 73034 (US)
(74) Representative: Gulde & Partner
(86) International application number: PCT/US2020/066048
(87) International publication number: WO 2021/127462

(56) References cited:
- WO-A1-2017/155858
- WO-A1-2019/219157
- WO-A1-2019/222657
- WO-A2-2014/028531
- WO-A2-2016/109604
- US-A1- 2016 186 239
- US-A1- 2018 288 982
- US-A1- 2018 288 982
- SINHA ET AL.: "Multiplexed real-time polymerase chain reaction cell -free DNA assay as a potential method to monitor stage IV colorectal cancer", SURGERY, vol. 166, no. 4, 1 August 2019 (2019-08-01), pages 534 - 539, XP085848309, DOI: 10.1016/j.surg.2019.06.004

## Description

### BACKGROUND

Cancer is one of the leading causes of death in developed, and increasingly also developing, nations. According to the World Health Organization, in 2012, over 14 million new cases were reported and over 8 million people died worldwide (Atlanta: American Cancer Society, Cancer Facts & 7Figures, 2014). Colorectal cancer (CRC) is the third most commonly diagnosed cancer and third-leading cause of cancer deaths in the United States. In 2014, nearly 140,000 diagnoses and 50,000 deaths are expected in the U.S. (Atlanta: American Cancer Society, Colorectal Cancer: Facts & Figures 2014-2016). CRC is often curable if detected early, and outcomes can be improved with post-treatment monitoring and surveillance for recurrence.

Effective cancer management depends on early diagnosis, accurate tumor staging, and consistent monitoring. While many methods have been developed for the detection and quantification of nucleic acids, e.g., NanoDrop Microvolume Spectrophotomer and Fluorometer, many current diagnostic procedures are invasive, expensive and unpleasant. In multiple recent published studies, circulating cell-free DNA (cfDNA) concentration and integrity (fragmentation pattern) has shown promise as a highly sensitive and specific, minimally invasive blood biomarker for multiple cancer types (*see, e.g.,* Hao, T B, et al., British Journal of Cancer 2014: 1-2, doi 10.1038/bjc.2014.470; Gonzalez-Masia, et al., Onco. Targets Ther. 6:819-832 (2013); Yu, J, et al., Lab Med. 45(1): 6-12 (2014)). A number of these studies have indicated the utility of a highly sensitive assay to measure cfDNA integrity (fragmentation pattern) and concentration based on quantitation of an ALU element, the most common type of retrotransposable elements (RE) in the human genome (Table 1). RE-based methods for quantitating DNA are attractive due to their superior sensitivity (multi-copy representation in the genome) and robustness. The sequence of ALU Yb8 and other ALU Yb subfamilies are known in the art, *see e.g.,* Ahmed et al., Mob DNA 2013, 4: 25 incorporated herein by reference in its entirety.

The most commonly employed cfDNA integrity/concentration assessment method, the ALU 247/115 bp index, targets sequences of a single ALU element, and thus the two fragments analyzed are not independent. This precludes use of these targets in a single multiplexed assay for maximum accuracy, efficiency and practical clinical use. This prior art method poses several particular problems. First, evaluating the first sequence and the second sequence in conventional single-plex polymerase chain reactions (PCR) wherein a single target is amplified in a single reaction well rather than multiplexing the two sequences into a single reaction mixture introduces well-to-well variability into the results. Every PCR reaction is somewhat different from every other PCR reaction, and experimental variation in set-up steps, such as variation in pipetting volumes, introduces error and can impact the results. Secondly, it has been shown (*see* U.S. Patent Application Publication No. US 2016/0186239 A1, published June 30, 2016, incorporated herein in its entirety) that the primers used in prior art studies to amplify these specific 247 bp/115 bp sequences show poor primer specificity, with false signals being generated from non-template controls. Thirdly, single-plex amplification prohibits the incorporation of an internal PCR control. The use of an internal PCR control is useful for confirming the success of the reaction and for providing confidence that other experimental factors such as the presence of PCR inhibitors in the sample have not interfered with sample integrity. Additionally, single-plex amplification of each target is cumbersome, more labor-intensive and less cost effective than is running a multiplexed amplification.

One of the cancer types studied using cell free DNA integrity is colorectal cancer (CRC). The current gold-standard for CRC diagnosis and staging is colonoscopy and subsequent histological examination. While specific and accurate, colonoscopy is invasive, expensive, and poses some risks; all of which decrease patient compliance to screening recommendations and discourage routine monitoring. In CRC and a few other cancer types, tissue biopsy is supplemented with detection of cancer protein biomarkers in blood serum, e.g. carcinoembryonic antigen (CEA). Such assays have the significant advantage of being minimally invasive and also do not require immediate localization of the tumor. Nevertheless, these assays suffer from limited sensitivity. CEA, one component of the current standard of care for CRC post-treatment monitoring, has relatively low sensitivity and specificity for early (stages I and II) and late (stages III and IV) disease (early: 36% sensitivity and 87% specificity; late: 74% sensitivity and 83% specificity) (Fakih, M. G.; Padmanabhan, A., Oncology 20(6): 579-587 (2006)). Given this performance, CEA is not recommended for CRC diagnosis according to the National Comprehensive Cancer Network guidelines for CRC (Ms-PSEE, Hunt, S., NCCN, Clinical Practice Guidelines in Oncology (NCCN Guidelines®) Colon Cancer, 2013).

Furthermore, imaging tests such as computerized tomography (CT) scans, bone scan, magnetic resonance imaging (MRI), positron emission tomography (PET) scan, ultrasound, and x-ray, among other radiological imaging, may be used to monitor disease progression and therapeutic effectiveness. Such tests have the downside of exposing patients to large amounts of radiation over the course of their treatment and while they are in remission, are costly, and may be burdensome.

### cfDNA: A Brief Overview of Biology and Physiology

Characterization of cell-free DNA (cfDNA), DNA found in circulation in human blood plasma and serum, has emerged as an exciting prospect for a new generation of blood-based tools for cancer detection, monitoring and surveillance. It is also an exciting prospect for monitoring minimum residual disease, therapeutic effectiveness, and disease progression. Nucleic acid circulation in human blood plasma was first reported in 1948 (Mandel P; Metais P., C.R. Acad. Sci. Paris 142: 241-243 (1948)). Leon, et al., (1977) were the first to report that mean cfDNA levels were significantly higher in the serum of patients with malignant cancers versus healthy patients (Leon, SA; Shapiro, B; Sklaroff, D M; Yarns, M J, Cancer Research 1977: 646-650). In the past two decades, many details of cfDNA biology, and the relationship between cfDNA and disease, have been elucidated. A brief primer of these studies is provided below.

Circulating cfDNA is derived from both the nuclear and mitochondrial genomes of normal and tumor cells (Mandel and Matais 1948, referenced supra; Zhong, S; Ng, M CY; Lo, Y MD; Chan, JC N; Johnson, P J; Kong H., J. Clin. Pathol. 53: 466-469 (2000)). Both coding and noncoding portions of the genome are represented among circulating cfDNA (Bettegowda, C, et al., Sci. Transl. Med. 6(224): 224ra24 (2014), doi: 10.1126/ scitranslmed.3007094.Detection). Although several mechanisms are believed to contribute to the circulating cfDNA pool, including spontaneous release of free, exosome encapsulated, and microvesicle-encapsulated DNA into the bloodstream, cell death is the major generator of circulating cfDNAs (Jahr, S; Hentze, H; Englisch, S; Hardt, D; Fackelmayer, F O; Hesch, R, Cancer Research 61:1659-1665(2001)). Cell turnover in normal cells is ordinarily due to apoptosis, which results in stereotyped sized fragments of DNA: a monomeric form composed of ~ 180 bp fragments of DNA and associated nucleosomes, and reduced amounts of oligomeric forms. *Id.* Alternatively, tumor cells turn over using a diversity of cell death pathways, not only apoptosis, but also necrosis, autophagy, and mitotic catastrophe (Jin, Z; El-Deiry, W S, Cancer Biology & Therapy 4(2): 139-163 (2005), available at http ://fly-bay.net/ journals/cbt/jin4-2.pdf (accessed 15 Dec. 2014)). Non-apoptotic pathways non-specifically and incompletely degrade DNA, generating substantially longer DNA fragments, up to 21 kilo bases in the case of necrosis (Jahr, S., cited *supra*)*.* Differences in the rate of cell death and type of cell death pathway utilized between normal and cancer cells lead to distinct characteristics of cfDNA pools that distinguish patients with and without cancer. cfDNAs have variable half-life within the body, ranging from minutes to hours (Lo Y MD; Zhang J; Leung TN; Lau T K; Chang AM Z; Hjelm NM, Am. J. Hum. Genet. 64: 218-224(1999); Emlen W; Mannik M., Clin. Exp. Immunol. 56(1): 185-192 (1984); Corcoran and Chabner, N Engl J Med 2018;379:1754-65). Short half-life implies that circulating cfDNA levels provide a dynamic measure of the physiological and pathological state of an individual. Finally, there is evidence that a small fraction of circulating cfDNA from blood is able to pass the kidney barrier and enter urine. These cfDNAs are called 'trans-renal' cfDNAs (Su Y-H, et al., J. Molecular Diagnostics, 6(2): 101-107 (2004); Botezatu I, et al., Clin. Chem. 46(8): 1078-1084 (2000)). The specific physiology of transrenal cfDNAs awaits detailed exploration.

Circulating cfDNAs from patients with and without cancer differ in a number of ways. Tumor genomes harbor specific genetic and epigenetic alterations that distinguish them from normal genomes, and these differences are reflected in cfDNAs. Nonspecific characteristics of cfDNA, such as concentration and integrity, differ between cancer patients and control subjects due to the specific mechanisms of cfDNA release into the blood by normal versus tumor cells. cfDNA concentration and integrity have often been found to be elevated in patients with cancer due to high rate of tumor cell death (reviewed in Schwarzenbach H; Hoon D S B; Pantel K., Nature Reviews Cancer 11: 426-437 (2011 ), doi: 10. 1038/nrc3066; Gonzalez-Masia, J A; Garcia-Olmo, D; Garcia- Olmo, D C, Onco. Targets. Ther. 6: 819-832 (2013)). However, absolute cfDNA concentration significantly varies among currently employed assays, significantly hampering the ability to compare results across studies. There is currently no standardized, validated, commercially available cfDNA concentration and integrity assay. There are no reports in the prior art of using a multiplexed quantitative polymerase chain reaction (qPCR) system of the kind described herein for accurate simultaneous measurement of concentration and integrity of cfDNA.

Efforts in cell-free DNA (cfDNA) testing using blood samples focus almost exclusively on mutations which are not tumor-type agnostic and lack the analytic sensitivity required for therapy monitoring. cfDNA was detected in blood as early as the 1940s by Madel and Metais (McLarty, J.L., Yeh, C.-H. Circulating Cell-Free DNA: The Blood Biopsy in Cancer Management. 2015 (cited 2020 Nov. 6); Available from: https: //circulogene. com/wp-content/uploads /2015/10/MOJCSR-02-00021.pdf.) and since this time, many studies have shown that the presence of elevated levels of cfDNA in the blood of patients with several cancer types including colorectal cancer (CRC) have poor prognosis (Bortner, et al., Trends Cell Biol, 1995. 5(1): p. 21-6.; Liu, X., et al., Enrichment of short mutant cell-free DNA fragments enhanced detection of pancreatic cancer. EBioMedicine, 2019. 41: p. 345-356). The elevation in cfDNA levels originates through the release of DNA from cellular necrosis and apoptosis of tumor cells. In healthy individuals, the main source of cfDNA in circulating blood is through necrosis. Necrosis generates a spectrum of DNA fragments of different sizes, due to random digestion by DNases (up to several kbp). Apoptosis generates small and uniform DNA fragments (less than <180bp) (Lapin, M., et al., J Transl Med, 2018. 16(1): p. 300). A recent study of fragment length of cfDNA shows mutant KRAS alleles tend to be significantly shorter when compared with DNA fragments bearing wild-type allele by densitometry in pancreatic cancer (Dasari, A., et al., Nat Rev Clin Oncol, 2020). Older publications often reached unreliable conclusions about correlation of cfDNA fragment size concentration and cancer progression due to inadequate cell free DNA purification protocols for small DNA fragments, as well as sample collection, plasma separation and storage protocols causing contamination of large DNA fragments from whole cell degradation. However, with the availability of new sample collection tubes with preservatives to prevent cellular contamination, such as Streck tubes, as well as availability of improved cfDNA extraction methods, several recent published studies have clearly established that cfDNA from cancer cells are smaller in fragment size as compared to cfDNA produced by noncancerous cells (Lin, S.Y., et al., JCO Precis Oncol, 2018. 2).

In clinical oncology, cfDNA has been suggested as a new surrogate marker for therapy response, disease progression and/or detecting early relapse. More recent studies have begun to explore the potential use of circulating tumor DNA (ctDNA) and oncogene biomarkers for prognostic uses (Elazezy, M. and S.A. Joosse, Comput Struct Biotechnol J, 2018. 16: p. 370-378.); Tie, J., et al., Ann Oncol, 2015. 26(8): p. 1715-22). Although ctDNA markers can provide great information in cancer biology, challenges and limitations have arisen when working with it as ctDNA can be as little as 0.01% of the entire cfDNA in plasma (Tie, J., et al., Ann Oncol, 2015. 26(8): p. 1715-22). Due to heterogeneity intratumorally as well as between tumors and metastatic lesions make it difficult to detect the cancer progression within individual patients. Additionally, the use of oncogene biomarkers may only represent a subpopulation of patients expressing these genes resulting in the missed opportunity to monitor an entire population of patients undergoing treatment. For ctDNA analysis, the use of sophisticated instrumentation by highly trained personnel, high blood volume requirements and cost are prohibitive factors especially in low resource areas and for economically disadvantaged patients. On the contrary, cfDNA is circulating in every individual's blood while elevated in cancer patients. A recent study comparing RECIST results to Carcinoembryonic Antigen (CEA), cfDNA or ctDNA levels demonstrated cfDNA had the highest correlation compared to RECIST for tumor burden and tumor volume of the main lesion (Henley, et al., Invasive Cancer Incidence, 2004-2013, and Deaths, 2006-2015, in Nonmetropolitan and Metropolitan Counties - United States. 2017 (cited 2020 Nov. 6); Available from: https ://www. cdc.gov/ mmwr/volumes /66/ss/ss6614a1 .htm ). This study highlights a great potential use of cfDNA for cancer monitoring, which can track the change in tumor burden.

### Overview of Retrotransposable Elements (REs)

Retrotransposable Elements (REs) are mobile element insertion polymorphisms that are essentially homoplasy-free characters, identical by descent and easy to genotype (reviewed in Batzer M A; Deininger, P L, Nat. Rev. Genet. 3(5): 370-9 (2002), doi:10.1038/nrg798). ALUs are REs that are approximately 300 bp insertions and are distributed throughout the human genome in large copy number. In addition to the major retrotransposon families, REs include smaller families of transposons such as SVA or long interspersed element ("LINE"). SVA elements, named after its main components, short interspersed element ("SINE"), variable number tandem repeat ("VNTR") and Alu element ("ALU"), contain the hallmarks of retrotransposons, in that they are flanked by target site duplications ("TSDs"), terminate in a poly(A) tail and they are occasionally truncated and inverted during their integration into the genome (Ono, M; Kawakami, M; Takezawa, T, Nucleic Acids Res. 15(21): 8725-8737 (1987); Wang, H, et al., J. Mol. Biol. 354( 4 ): 994-1007 (2005), doi: 10.1016/j jmb.2005.09.085). Long-interspersed Elements (LINE) are similar to ALU and SVA in that they also contain the hallmarks of retrotransposons and are high copy number, but differ in size, being up to several kilo bases in length (Deininger, P L; Batzer, M A, Genome Res. 12(10): 1455-65 (2002), doi:10.1101/ gr.282402).

### RE-Based DNA Quantitation

RE-based quantitation methods are advantageous when compared to current, commercially available systems due to the presence of a large number of fixed insertions. With a high copy number of subfamily-specific RE repeats within the human genome, these human-specific DNA assays have a very sensitive dynamic range of 1 pg to 100 ng (Nicklas, J A; Buel, E., J. Forensic Sci. 48(5): 1-9 (2003)). For example, the ALUYb lineage contains approximately 1800 copies per genome and SVA contains approximately 1700 full length element copies per genome (Wang, H., referenced *supra*; Carter, A B, et al., Hum. Genomics 1(3): 167-178 (2004)). This large copy number minimizes the effect of variation between individuals, resulting in highly reproducible quantitation values.

U.S. Patent Publication 2014/0051075 A1, to Sudhir K. Sinha, is entitled "Development of a Highly Sensitive Quantification System for Assessing DNA Degradation and Quality in Forensic Samples" and describes the detection of DNA quality with a multiplex reaction using ALU and SVA for human DNA quantification. Though very useful for forensic purposes, the described method does not detail specific application to cell free DNA from plasma and/or serum. The amplicon sizes needed for a cfDNA assay are different from those needed for forensic applications, and other details of the two methods such as amplification conditions and primer/probe concentrations differ as well.

Exemplary embodiments of prior art methods and systems for multiplex determination of cfDNA in a sample are disclosed in WO 2016/109604 A2, US 2018/288982 A1 and WO 2014/028531 A2.

### SUMMARY OF THE INVENTION

A majority of healthy (non-cancer) human cfDNA fragment sizes are around 140-180bp long. Cell free DNA released from cancer cells (often called circulating tumor DNA or ctDNA) are shorter than the cfDNA released from normal cells. In contrast to cfDNA in samples from cancer patients, the majority of cfDNA fragments from non-cancer humans are generated from apoptotic cells, generating around 180 bp-long (or 140-180bp) fragments equivalent to the length of DNA that wraps around 1 nucleosome, and sometimes accompanied by DNA fragments with sizes in multiples of 180 bp. The qPCR method of measurement of any retrotransposable element (RE) target sequence quantitates cfDNA fragments equal to or longer than the size of the RE target sequence. For example, an qPCR measurement of Yb8 ALU target sequence of 80bp quantitates cfDNA fragments of >80bp in length, including both short and long cfDNA fragments that comprise the 80 bp RE target sequence. On the other hand, qPCR measurement of a 265bp SVA target sequence quantitates cfDNA fragments of >265bp, those comprising the 265 bp RE target sequence, which does not include cfDNA fragments of less than 265bp in length

The present invention is directed to subject matter as defined in the appended claims.

Herein we discuss the development and evaluation of a retrotransposable interspersed element (RE) based multiplexed qPCR assays to robustly quantitate and distinguish cfDNA integrity and concentration in test and control subjects' bodily fluids, e.g., plasma and serum. The assays provide accurate, minimally invasive, rapid, high-throughput, and cost-effective methods with the potential to complement for characterizing minimum residual disease, therapeutic effectiveness, and disease, e.g., cancer, progression in humans, thereby improving patient outcomes.

The methods described herein for assessing the cfDNA and ctDNA integrity and concentration and thereby assessing the presence of cancer cells do not depend on a clonal mutation being present in the cancer cells. As such, the methods are "agnostic" in that the methods can be applied to samples from patients having many different types of cancers. Moreover, the sensitivity of the methods described herein is far greater than other cfDNA and ctDNA assays as the levels of cfDNA and ctDNA above a normal threshold are detected in virtually all cancer patients tested. In addition, the methods described herein have low Cost of Goods Sold, are based on commonly used qPCR lab methodology and have a fast Turnaround Time (TAT), e.g., the DNA integrity and concentration can be assayed and a conclusion as to the presence of cancer cells or if a cancer therapy is ineffective and whether a patient has progressive disease can be completed quickly, e.g., in less than 24 hours, less than 18 hours, less than 12 hours, or less than about 4 hours.

An embodiment of the invention is a method whereby RE targets are simultaneously assayed in a single, highly sensitive qPCR reaction, wherein a single RE target is amplified in a single qPCR reaction vessel, e.g., a well, (singleplex qPCR) or wherein multiple RE targets are amplified in a single qPCR reaction vessel, e.g., a well (multiplex qPCR) , optionally including an internal positive control to monitor the presence of PCR inhibitors potentially present in the sample of blood serum, plasma, urine, or other biological fluid. This method enables development of an accurate, rapid, affordable, minimally invasive, high throughput, cost effective clinical test to complement or replace existing procedures and improve characterizing minimum residual disease, therapeutic effectiveness, and disease progression in humans.

Accordingly, one embodiment of the invention is a qPCR method that accurately quantitates cfDNA in a patient's biological fluids including, e.g., blood plasma or serum as an indication of cancer cells present in the patient or as an indication of the ineffectiveness of a neoadjuvant or a cancer therapy or as an indication the patient has progressive disease. The method may be singleplex wherein a single RE target is amplified in a single qPCR reaction well or the method may be multiplex wherein multiple RE targets are amplified in a single qPCR reaction well.

Another embodiment of the invention is a qPCR method that accurately provides a determination of the extent of fragmentation or integrity of cfDNA in biological fluids including, e.g., blood plasma or serum, as an indication in the level of "minimum residual disease" ("MRD"). The method may be singleplex wherein a single RE target is amplified in a single qPCR reaction well or the method may be multiplex wherein multiple RE targets are amplified in a single qPCR reaction well.

It is also disclosed a three RE target (a first "short" RE target, a second "short" RE target, and one "long" RE target) multiplex RE-qPCR assay to accurately and robustly obtain cfDNA concentration, a determination of fragmentation and integrity, and DNA integrity index ("DII" or "DI") of biological samples from normal controls and patients having cancer, e.g. colorectal cancer (CRC), by direct qPCR from plasma or serum samples with or without DNA purification. The assay may also include one internal positive control synthetic target. The short RE targets are preferably about 60bp to about 135bp in length, about 70 bp to about 130bp, or about 60 to about 120 bp in length with the proviso that the short RE targets differ sufficiently, e.g., in length and sequence, so that their amplification products generated in the qPCR assay can be distinguished from each other, e.g., the short RE targets may differ at least by about 10bp, at least by about 15 bp, or at least by about 20 bp in length. The third long RE target is preferably about 200 bp to 300 bp in length, or 207 bp to 270 bp, e.g., about 260 bp to 267 bp. DII indicates a level of cfDNA fragmentation and is a ratio of long target quantities to a short target quantity. DII as used herein is a ratio of the long RE target, e.g., 265 base-pairs to the short RE targets, e.g., 80 base-pairs (265 bp/80 bp). When DII (265 bp/80 bp) is lower than 0.4, it indicates the major source of cfDNA is from apoptotic cells. When DII (265 bp/80 bp) is above 0.4, cfDNA are also generated through necrosis. Information on DNA integrity and DII, is found in Sinha et al., Surgery, DOI: https :// doi. org/10.1016/j.surg.2019.06.004 (2019) and Madhavan, Dharanija, et al., Epidemiology, DOI 10.1007/s10549-014-2946-2 (2013) incorporated herein in their entirety by reference.

An embodiment of the invention is a multiplexed method to quantitate the integrity of circulating cell free human DNA in a test subject, comprising providing a sample of serum, plasma, urine, or other biological fluid from the test subject, the sample comprising cell free human DNA, and the cell free human DNA comprising a first and second short RE target each having a length of between about 60 and 135bp, about 70 to about 130 bp or about 60 and 120 base pairs, then using a multiplex quantitative polymerase chain reaction (qPCR) method to quantitate the short RE targets, obtaining for the quantitated RE targets a threshold cycle number, comparing each threshold cycle number with a standard curve to determine a quantity of the RE targets that was present in the sample, and determining the quantity of each of the RE targets is higher in the test subject's sample as compared to a control sample, e.g. a sample from a healthy subject, concluding the test subject should receive a treatment and administering the treatment to the test subject.. For example, the cfDNA concentration measured for a first short RE target of 80 bp (Yb-8-80bp), and a second RE target of 120 bp (Yb-8-120bp), and a third RE target of 265 bp (SVA 265), in plasma samples from 40 healthy controls and 39 cancer patients is set forth in Table 1. The RE targets were amplified using the primer pairs for Yb-8-80bp, Yb-8-120bp and SVA 265 set forth in Tables 2A and 2B. The data in Table 1 demonstrate that while the absolute levels of the retrotransposable element targets are all different in each sample the amount of cfDNA in cancer patients is greater than that in control subjects. The concentration of the shortest 80 bp target is consistently higher than the longer the 120 bp target and the 265 bp target indicating that the cfDNA is highly degraded (apoptotic cell death).. The method may further comprise the step of concluding the subject is in need of a cancer therapy or has progressive disease based on the difference in the amount of the short targets being above the threshold amount in a control sample, and optionally also based on the DII of the sample, and then administering the treatment to the subject.

It is further disclosed a multiplexed method to quantitate the integrity of circulating cell free human DNA, comprising providing a sample of serum, plasma, urine, or other biological fluid, preferably a plasma sample, the sample comprising cell free human DNA, the cell free human DNA comprising two retrotransposable element (RE) targets, a short RE target sequence between 60bp and 135 base pairs or between, 60bp and 120 base pairs, or about 70bp to about 130bp, and a long RE target sequence between 200bp-300bp, about 207bp to about 270bp, or about 260 bp to about 265 base pairs, the retrotransposable element genomic targets are preferably independent of each other, using a multiplex quantitative polymerase chain reaction (qPCR) method to separately and simultaneously quantitate the short and long RE targets, obtaining for each quantitated RE target a threshold cycle number, comparing each threshold cycle number with a standard curve to determine for each quantitated RE target a quantity of the RE targets that were present in the sample, and (i) calculating a ratio of the quantity of the long RE target to the quantity of the short RE target, and concluding based on the long RE target/short RE target ratio the subject should receive a treatment and administering the treatment to the subject.

It is further disclosed a multiplexed method to identify a subject who has cancer or MRD comprising,
providing a sample of serum, plasma, urine, or other biological fluid, from the subject, the sample comprising cell free human DNA, the cell free human DNA comprising (a) a first short RE target and a second short RE target, the short target being between about 60 and about 135 base pairs, between about 70bp and about 130bp, or between about 60 bp and about 120 bp in length with the proviso the first and short RE targets differ in size and (b) a long RE target being between about 200bp and about 300 bp in length,
using a quantitative polymerase chain reaction (qPCR) method to quantitate the first and second short targets in the sample and obtaining for each of the quantitated targets a threshold cycle number,
comparing each threshold cycle numbers with a standard curve to determine the amounts of the RE targets that were present in the sample,
determining the difference in the amounts of the two short RE targets,
identifying the subject as having cancer or MRD by determining the difference between the quantity of the two short RE targets is increased as compared to a control. The method may further comprise determining the DII for the DNA in the sample. The method may further comprise administering an appropriate treatment to the subject identified as having cancer or MRD.

It is further disclosed a multiplexed method to identify a neoadjuvant or cancer therapy as ineffective or identify a subject who has a progressive cancer, is in remission, or has MRD comprising,
providing a first sample of serum, plasma, urine, or other biological fluid, taken from a subject before administering a cycle of cancer therapy and a second sample of serum, plasma, urine, or other biological fluid from the subject after the cycle of cancer therapy, the first and second samples comprising cell free human DNA, the cell free human DNA comprising (a) two short RE target between about 60 and about 135 base pairs, between about 70bp and about 130bp, or between about 60 bp and about 120 bp, and (b) a long RE target between about 200 bp to about 300 bp, between about 207 to about 265 bp, between about 260 to about 265bp in length
using a quantitative polymerase chain reaction (qPCR) method to quantitate the two short RE targets and the long RE target in the first and second samples and obtaining for the quantitated short and long RE targets a threshold cycle number,
comparing each threshold cycle number with a standard curve to determine the amounts of the short and long RE targets that were present in the first and second samples,
determining the difference between the quantity of the short targets is increased in the second sample
identifying the subject having an increased the short RE targets in the second sample as having received an ineffective neoadjuvant or cancer therapy or as having a progressive cancer or MRD. In this method the samples may be obtained from the subject one week apart, at least 2 weeks apart, at least 3 weeks apart or at least 4 weeks apart, e.g., 12 to 21 days apart, provided the second sample is obtained from the subject before the next cycle of therapy. The steps of this method may be repeated throughout multiple cycles of therapy, wherein a sample is obtained before and after each cycle of therapy and subjected to this method. The method may further comprise calculating a DII of the DNA in the samples. The method may further comprise administering an appropriate treatment to the subject identified as having the progressive cancer or MRD.

In the multiplexed method to identify a cancer therapy as ineffective or identify a subject who has a progressive cancer, the difference between the quantity of the short RE targets in each of the first and second samples may be determined by determining the value of the amount of the shorter of the short targets minus the amount of the other short RE target in the first sample (said value = Frag 1) and determining the value of the amount of the shorter of the short targets minus the amount of the other short RE target in the second sample (said value = Frag 2) and determining the value of Frag1 minus Frag2 (said value = FragDiff) wherein a FragDiff of greater than a threshold value identifies the cancer therapy as ineffective or the subject as having progressive cancer or MRD.

In certain embodiments of the multiplexed method of the present invention, the retrotransposable element genomic targets may be an interspersed ALU, SVA or LINE1 element. In certain embodiments of the multiplexed method of the present invention, the retrotransposable element genomic targets may be each independently an interspersed ALU, SVA, or LINE element. In certain embodiments, these retrotransposable element genomic targets may each have a copy number in excess of 1000 copies per genome.

Some embodiments of the multiplexed method of the present invention further comprise a step of adding a synthetic DNA sequence to the sample as an internal positive control (IPC) prior to the using step/ qPCR quantitation step, quantitating the internal positive control in the using step, and utilizing the quantitative internal positive control result in the comparing step to improve the accuracy and reliability of the comparing step to determine the amounts of the RE targets.

In embodiments of the multiplexed method of the present invention, the use of an internal positive control enables a determination of the concentration of cell free DNA in the sample.

In some embodiments of the multiplexed method of the present invention, the sample of serum, plasma, urine, or other biological fluid may be placed in a single tube, and the qPCR reactions for quantitation of the nucleic acid fragments may be carried out in that same single tube. Alternatively, each nucleic acid fragment may be separately and simultaneously amplified in separate tubes.

In some embodiments of the multiplexed method disclosed, the ratio of the quantity of the longer RE target to the quantity of a shorter RE target may serve as the DII of circulating cell free DNA for diagnostic and therapeutic applications. These diagnostic applications may include one or more of the characterizing minimum residual disease, therapeutic effectiveness, and disease progression in human patients, and treating such patients.

In certain embodiments, the multiplexed method of the present invention may include a step of deactivating or eliminating proteins that bind to the short nucleic acid fragment or the long nucleic acid fragment. This may be done by mixing the sample with a buffer including a surfactant and chelating agent, enzymatically digesting the protein, then using heat to deactivate and inactivate the digested protein, followed by centrifugation. Alternatively, dilution of the sample using 40 parts sterile water to one part sample by volume may have the effect of deactivating or eliminating these proteins.

In some embodiments, the multiplexed methods of the present invention may include a step of separating amplification products obtained from the qPCR reaction using electrophoresis. In some embodiments of this invention, the amplification products of the qPCR method used in the methods of this invention may be detected and/or quantified using electrical biosensors (see Liu, et al., Single-Nucleotide Polymorphism Genotyping Using a Novel Multiplexed Electrochemical Biosensor with Nonfouling Surface. Biosens. Bioelectron. 2013, 42, 516-521).

In some embodiments, the multiplexed methods of the present invention may include a step of determining an optimum temperature for the qPCR reaction.

The multiplexed methods of the present invention may include a sample that comes from an individual who is suffering from cancer, is in remission from cancer, or who is at risk for developing cancer, who has received a treatment for cancer, e.g., a targeted therapy, chemotherapy, immunotherapy, targeted-immunotherapy, surgery to remove a tumor, or a radiotherapy. Targeted therapy is a type of cancer treatment that uses drugs or other substances to precisely identify and attack certain types of cancer cells. A targeted therapy can be used by itself or in combination with other treatments, such as traditional or standard chemotherapy, surgery, or radiation therapy.

In certain embodiments, the present invention may take the form of a multiplexed system for evaluating the effectiveness or ineffectiveness of a cancer therapy or for characterizing cancer in humans, the system including a sample of serum, plasma, urine, or other biological fluid, preferably a plasma sample, the sample comprising cell free DNA. The cell free DNA comprises one short retrotransposable element targets, or two short retrotransposable element targets, and optionally a long retrotranspoable element target. The short retrotransposable element targets may have a length in the range of about 60 base pairs to about 135 base pairs, about 70 to about 130bp, or 60 base pairs to about 120 base pairs, and two short retrotransposable element targets, may each with a length of 60 base pairs to 135 base pairs, or about 70 bp to about 130 bp, or about 60 base pairs to about 120 base pairs, preferably the two short RE targets differ in size and sequence sufficiently to distinguish their amplification products generated in the qPCR assay, e.g., the short RE targets differ in size by at least about 10 bp, at least about 15 bp, or at least about 20. The third RE target being a fragment of another multi-copy retrotransposon with a length of about 200 bp to about 300bp, e.g., 207 bp to 265 base pairs. In an embodiment, the retrotransposable element targets are independent of one another. The system may further comprise an internal positive control (IPC) comprising synthetic DNA, a TaqMan^{®} probe corresponding to each RE target and IPC, each probe comprising a detectable label that is distinct from the labels incorporated into the other probes, a forward primer and a reverse primer pair for amplifying each RE target and IPC, a DNA standard for generating standard curves for each RE target and IPC, a qPCR system for simultaneously amplifying each RE target and IPC and for producing a threshold cycle number for each RE target and IPC, and a qPCR data analysis system for producing DNA quantitation values for each RE target by interpolation using threshold cycle numbers and linear standard curves and for using the DNA quantitation values to produce an indication of the integrity of the cell free DNA and for characterizing cancer in a human.

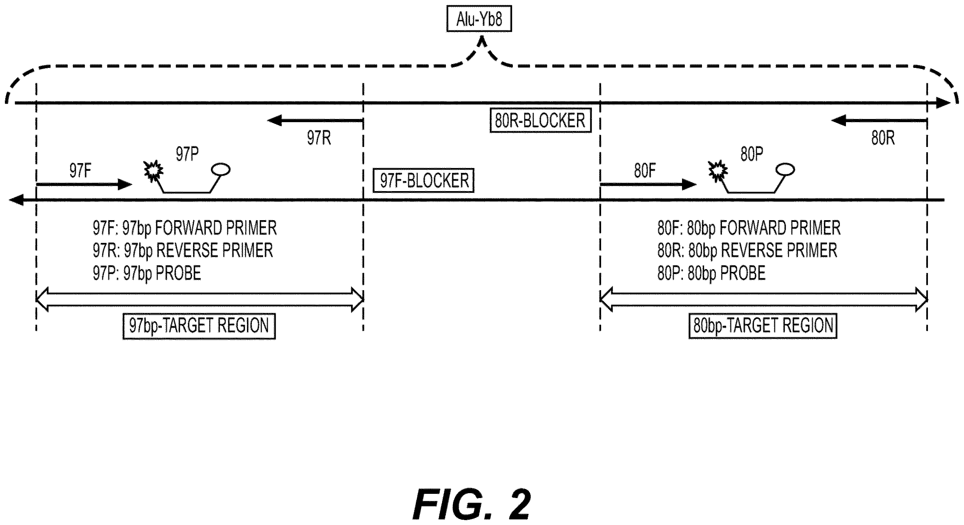

In one embodiment, one or more of the retrotransposable element targets used in the methods and systems of the invention described herein, are an ALU (e.g. ALU-Yb8) target or an SVA. The ALU target may be, for example, a 60 bp target, a 65 bp target, a 71bp target, an 80 bp target, a 97 bp target, a 105 bp target. The targets may be amplified with forward and reverse primers. In embodiments comprising multiple RE targets from the same RE, e.g., ALU Yb-8, , PCR blockers/PNA clamping may be included to limit extension from the primers beyond the position of the blockers, thus limiting the extension from a primer pair used to amplify one RE target into the other RE target and thereby enhancing the specificity by limiting the production of extraneous or overlapping products. Preferably the PCR blockers are peptide nucleic acid (PNA) oligos and bind to the retrotransposable element between the targets to be amplified. See e.g., Figure 2 depicting the position of the forward and reverse primers used to amplify an 80 base pair target and 97 bp target on an ALU, e.g., ALU-Yb8, and the position of the 80 bp blocker that limits extension from the 80 bp forward primer, and a 97 bp blocker that limits extension from the 97 bp blocker. In this embodiment, during qPCR the 80 base pair and 97 base pair specific forward primer and reverse primer hybridize to their respective sites on the ALU, but extension from the primers is limited by the presence of the PCR blocker at their respective sites.

### BRIEF DESCRIPTION OF THE FIGURES

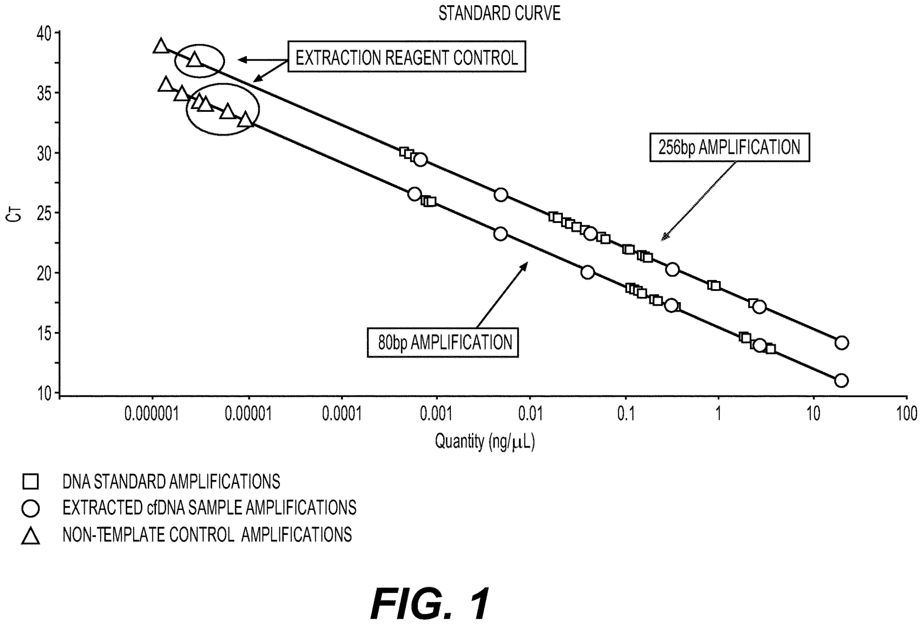

Figure 1. Titration illustration using a standard curve of known quantities of cfDNA with concentration measured in ng/µL using the test described in US Patent Application Publication No. US 2016/0186239 A1, incorporated by reference herein in its entirety. The x-axis depicts the concentration of DNA fragments longer than 80 bp and longer than 265 bp measured in ng/µL. The y-axis depicts the number of the PCR amplification cycle.
Figure 2. Diagram showing two PCR target regions, 80 bp and 97 bp, on the Alu-Yb8 sequence using two peptide nucleic acid (PNA) oligos to block PCR extension beyond the target regions.
Figures 3A and 3B present the Log-odds (y-axis) vs Frag1(x-axis) (FIG. 3A) and Log-odds (y-axis) vs FragDff (ng/ml)(x-axis) (FIG. 3B).
Figure 4 is an illustration of the specificity of a method described herein used in identifying samples from patients with progressive disease.

### DETAILED DESCRIPTION OF THE INVENTION

There is a clear need in cancer management, and colorectal cancer (CRC) treatment specifically, for a standardized and validated blood test to sensitively and robustly quantitate cfDNA integrity and concentration. The present application addresses this need by creating a multiplex qPCR assay for quantitating cfDNA integrity and concentration based on retrotransposable element targets to identify, characterize and/or appropriately treat the patient having cancer, e.g., progressive disease, or MRD. The assays are also useful in indentifying a cancer therapy's effectiveness or ineffectiveness.

The most commonly employed method conducted by others in the field of cfDNA integrity and concentration assessment for cancer detection and monitoring is qPCR using the ALU 247/115 index. The methods described herein for assessing integrity and concentration of cfDNA and ctDNA quantitates "short" retrotransposable element targets having lengths between 60bp and 135bp, 70 bp to 130bp, or between 60bp and 120 bp to reliably indicate therapy effectiveness or ineffectiveness. The ranges between 60bp and 135bp, between 70 to about 130bp, e.g., 71bp to 132bp, or between 60bp and 120 bp ranges of ALU, SVA and LINE1 retrotransposable elements targets are also useful for discriminating between normal (non-cancer) human and humans with cancer, particularly progressive disease, or the presence of MRD. Preferably the retrotransposable elements are ALU, e.g., Yb-8 ALU, SVA, or LINE1.

MRD refers to the small number of malignant cancer cells that remain in the body during or after treatment (see NCI Dictionary of Cancer Terms, https://www.cancer. gov/publications/dictionaries/ cancer-terms/def/797386). Even when a patient is in remission from cancer and the solid tumor has shrunk beyond detection, the patient may still have MRD. The MRD assessment is used to determine if additional treatment is necessary, if a treatment already administered has been effective in reducing tumor load, or to select and administer a particular treatment of the subject. MRD assessment is mainly used in blood cancers (leukemia, lymphoma and myeloma), but is being studied in other solid cancers. MRD assessment has been used in guiding the treatment of cancer patients in cases of, e.g., resected hepatoma, resection of mastectomy, esophageal cancer, rectal cancer, anal cancer, head and neck cancer, colon cancer, lung cancer, breast cancer, neu metastatic breast cancer.

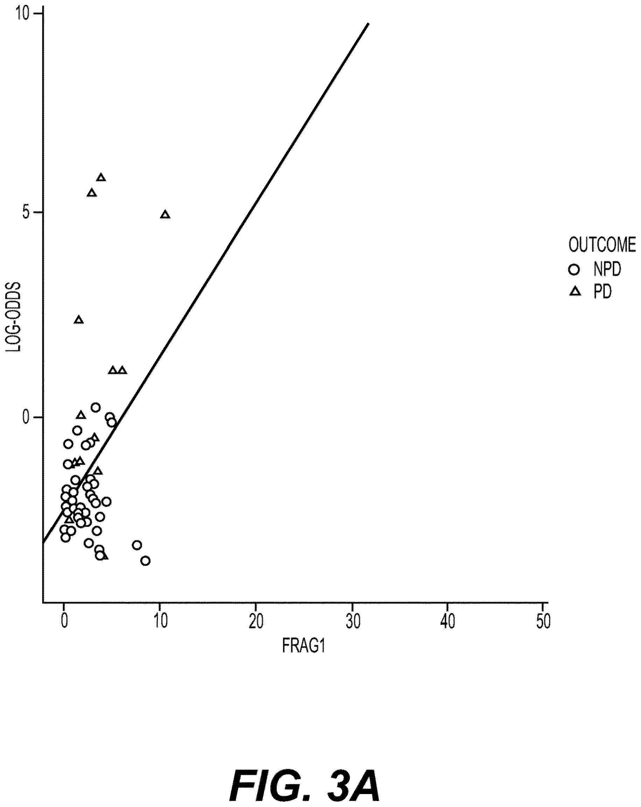

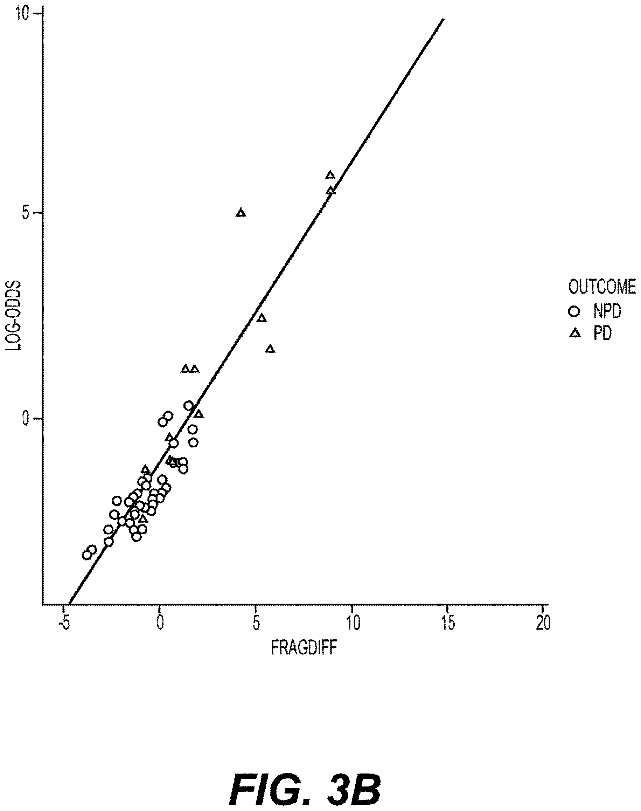

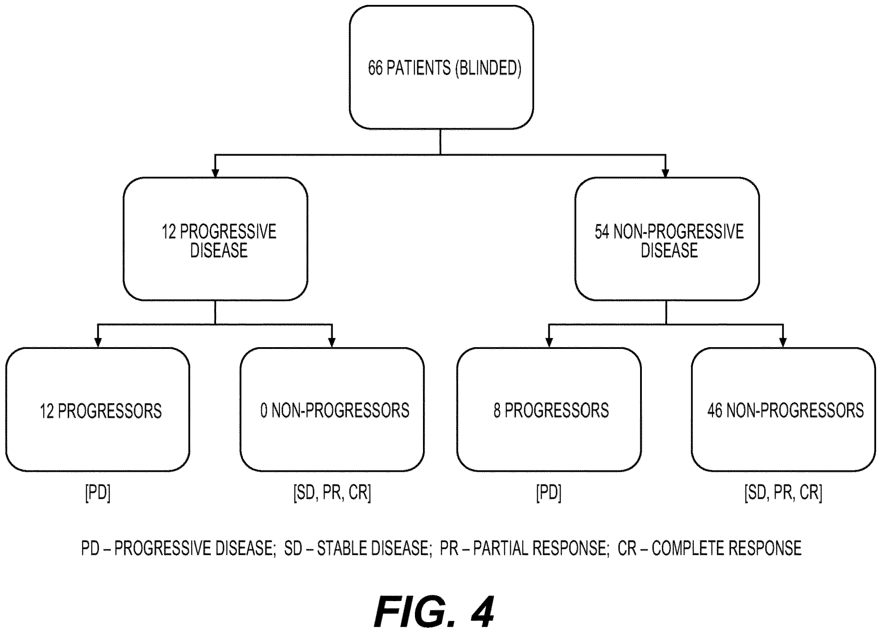

Cancer patients in remission must undergo quarterly imaging (e.g. MRI, x-ray, CT scan, or other radiology studies) to determine whether the cancer has returned. However, some patients in remission may not have a solid tumor that is detectable by imaging studies, but may still have MRD. The methods described herein for quantitating the integrity and concentration of cfDNA by using short retrotrasposable elements target(s) having a length between 60bp and 135bp, 70bp to 130bp, or 60 to 120bp, may be used to characterize cancer or MRD. The change in the amount of the quantitated short RE target sequence between 60bp and 135bp, 70 bp to 130bp or 60 bp to 120 bp over time may be used alone or in conjunction with standard assays to reliably identify subjects who have MRD or cancer progression or evaluate the ineffectiveness of a cancer therapy. Based upon a determination that the subject has MRD or progressive cancer, or the ineffectiveness of a therapy, additional rounds of therapy or another therapy may be administered to the subject. We demonstrate herein that cfDNA comprising elevated or increasing amounts of short ALU Yb8 targets of 60 base pair to 135 base pair, about 70bp to about 130bp, or 60bp to about 120bp sequence as compared to the amount of long RE targets, e.g., SVA or LINE targets, between 200 bp and about 300 bp, or between 207 bp and about 270 bp, between 260bp and 265bp, e.g., 265bp or 267 to be highly effective in discriminating between normal humans (non-cancer) and humans with cancer (see e.g., Figure 3 and Figure 4). And because the methods herein do not rely on detecting CEA, the methods are "agnostic" and can be applied to samples from patients having or suspected of having any type of cancer, e.g., colorectal cancer (CRC), hepatoma, esophageal cancer, rectal cancer, anal cancer, head and neck cancer, colon cancer, lung cancer, e.g., non-small cell lung cancer (NSCLC), small cell lung cancer (SCLC) breast cancer, and blood cancers, e.g., leukemia.

The methods described herein for assessing cfDNA integrity and concentration, using a sample from a subject, *e.g.,* a plasma or serum sample or another bodily fluid sample, and RE targets, are useful in detecting, measuring, or monitoring cancer and are an additional parameter for use in the assessment of tumor load, cancer progression, therapy ineffectiveness and or MRD such that an appropriate treatment is administered to the subject. The methods described herein allow for detection of cancer cells in patients who have a nearly undetectable level as determined by standard clinical tests, such as imaging assays, e.g., CT scans or Xrays, or detection of cancer cells in a blood or tissue sample. The patients may be a patient suspected of having or treated for hepatoma, esophageal cancer, rectal cancer, anal cancer, head and neck cancer, colon cancer, colorectal cancer (CRC), lung cancer, e.g., non-small cell lung cancer (NSCLC), small cell lung cancer (SCLC) breast cancer, and blood cancers, e.g., leukemia. Thus, the methods described herein are an improvement over existing methods because they reduce patients' exposure to radiation from imaging studies.

Patients diagnosed with cancer, including patients receiving a cancer therapy, may be categorized based on their disease progression, e.g., following a cycle of chemotherapy or immunotherapy or other therapeutic regime. A "complete response" ("CR") patient is one where there is no evidence of the disease due to a disappearance of all target lesions as determined by standard methods, e.g., such as CT scans or detection of cancer cells in a blood or tissue sample. A "stable disease" ("SD") patient is one where there is neither sufficient shrinkage of cancer lesion size to qualify for partial response ("PR") nor sufficient increase in lesion size to qualify for "progressive disease" ("PD") using as a reference the smallest sum of diameter of target lesions. A PR patient is one who demonstrates at least a 30% decrease in the sum of the diameters of target lesions vs. the baseline sum of the diameters of the target lesions. Additionally, the sum of the diameters of the target lesions must demonstrate an absolute increase of at least 5 mm or one or more new lesions have been detected to be considered PR. A PD patient is one where there is at least a 20% increase in the sum of the diameters of the target lesions vs. the smallest sum of target lesions, which may be the baseline sum.

The present invention is non-invasive and may also be used for screening high risk patients for onset of cancer, e.g., hepatoma, esophageal cancer, rectal cancer, anal cancer, head and neck cancer, colon cancer, colorectal cancer, lung cancer, breast cancer, neu metastatic breast cancer and blood cancers, e.g., leukemia. Patients may be considered "high risk" for a variety of reasons including past family history of cancer, environmental exposure, and lifestyle. However, it is not feasible, highly wasteful, and harmful for patients to be exposed to radiological scans to screen them for cancer.

The present invention may be used to distinguish between therapy ineffectiveness or futility and therapies that are partially ineffective. Current methods make it burdensome, costly, and inefficient to determine whether a therapy is ineffective in a patient or the patient experience a partial response to a therapy. The present invention allows clinical providers to detect noninvasively and quickly whether the therapy is entirely ineffective or partially ineffective. This allows providers to make quicker and better informed clinical decisions about patient therapy and administer an appropriate therapy.

One drawback of many currently available methods is the inability to identify cell necrosis. One method for identifying cell necrosis is the DII. DII is a ratio of long fragments quantities to short fragment quantities. DII indicates a level of cfDNA fragmentation. When the DII using the ratio of 265bp to 80bp targets is calculated and determined to be lower than 0.4, it indicates the major source of cfDNA is from apoptotic cells. When the DII using the ratio of 265bp to 80bp targets is calculated and determined to be above 0.4, cfDNA are also generated through necrosis. This DII may be used in the methods of this invention to assess cell necrosis.

Described herein are methods and systems for quantitating the integrity of circulating cell free human DNA and implementing a treatment of a patient. Disclosed herein is a method for quantitating the integrity of circulating cell free human DNA and implementing a treatment of a patient comprising:
(a) providing a sample of a bodily fluid comprising cell free human DNA, the cell free human DNA comprising an RE target of between 60 base pairs to 135 base pairs, about 70 to about 130bp, or 60 to about 120 bp in length;
(b) using a quantitative polymerase chain reaction (qPCR) method to quantitate the RE target;
(c) obtaining for the quantitated RE target a threshold cycle number;
(d) comparing the threshold cycle number with a standard curve to determine a quantity of the RE target that was present in the sample; and
(e) determining the quantitated RE target amount in the patient sample is higher than present in a control subject, and concluding the patient is in need of a treatment, and implementing the treatment of the patient. The method may be singleplex wherein a single RE target is amplified in a single qPCR reaction well or the method may be multiplex wherein multiple RE targets are amplified in a single qPCR reaction well.

The bodily fluid samples used in the methods of this invention should be treated so as to remove cells. Suitable bodily fluids include, e.g., serum, plasma, urine, saliva, tears or other biological fluid. Preferably the sample used in the methods and system of this invention is a plasma sample.

In the methods of this invention two or more short retrotransposable element targets of between 60 to 135bp, about 70 to about 130 bp or 60 bp to about 120 bp, may be subjected to the quantitative polymerase chain reaction (qPCR) method to quantitate the targets.

The methods of this invention may further comprise a step of adding a synthetic DNA sequence to the sample as an internal positive control (IPC) and quantitating the retrotransposable element targets and the IPC, and utilizing the quantitative IPC result in the step of comparing the qPCR threshold cycle numbers to a standard curve to improve the accuracy and reliability of the comparing step. The IPC also enables a determination of a concentration of cell free DNA in the sample when quantitating the RE targets by qPCR in a single tube.

The methods of this invention may further comprise a step of adding a hybridization probe that hybridizes to the RE targets to detect the targets. The probe may be added to the sample before the target(s) are subject to q-PCR or thereafter. The probe may include an observable label. Any observable label routinely used in the art for labeling nucleic acid probes could be used to label the probe, e.g., a fluorescent label. Suitable fluorescent probes include, *e.g.,* FAM, Cy5, Hex, or Cy3). The observable label may be detected using a microfluidic device.

The retrotransposable elements of the methods of this invention include e.g., an ALU, particularly ALU Yb8, an SVA, or a LINE element. The retrotransposable element may have a copy number in excess of 1000 copies per genome.

In the methods of this invention the short retrotransposable element targets may have a length from about 60 base pairs to about 135 base pairs, about 60 base pairs to about 120 base pairs, about 60 base pairs to about 120 base pairs, and about 70 bp to about 130 bp,. For example, the retrotransposable element target may have a length of e.g. 60bp, 65bp, 71 bp, 80 bp, 97 bp, 105 bp, or 120 bp. The RE targets may be amplified with the forward and reverse primer pairs set forth in Table 2A, and/or 2B:

| Table 2A : ALU-Yb8 targets' primer and probe sequences | | | | |
|---|---|---|---|---|
| Name | Size | Primer Type | Primer & Probe Sequence | SEQ ID NO |
| Yb8-80bp | 80bp | Forward | GGAAGCGGAGCTTGCAGTGA | 1 |
| | | Reverse | AGACGGAGTCTCGCTCTGTCGC | 2 |
| | | Probe | AGATTGCGCCACTGCAGTCCGCAGT | 3 |
| Yb8-71bp | 71bp | Forward | CTTGCAGTGAGCCGAGATT | 4 |
| | | Reverse | GAGACGGAGTCTCGCTCTGTC | 5 |
| | | Probe | ACTGCAGTCCGCAGTCCGGCCT | 6 |
| Yb8-97bp | 97bp | Forward | GTGGCTCACGCCTGTAAT | 7 |
| | | Reverse | GGGTTTCACCTTGTTAGCCA | 8 |
| | | Probe | TGGATCATGAGGTCAGGAGAT | 9 |
| Yb8-105bp | 105bp | Forward | AGGCAGGAGAATGGCGTGAACC | 10 |
| | | Reverse | AGACGGAGTCTCGCTCTGTCGC | 11 |
| | | Probe | AGATTGCGCCACTGCAGTCCGCAGT | 12 |
| Yb8-119bp | 119 | Forward | AGACCATCCTGGCTAACAA | 13 |
| | | Reverse | GCCATTCTCCTGCCTCA | 14 |
| | | Probe | | |
| Yb8-120bp | 120bp | Forward | TGGATCATGAGGTCAGGAGAT | 15 |
| | | Reverse | CCGAGTAGCTGGGACTACA | 16 |
| | | Probe | ACCATCCTGGCTAACAAGGTGAAACC | 17 |
| Yb8-123bp | 123bp | Forward | ATCCTGGCTAACAAGGTCAAA | 18 |
| | | Reverse | CGGGTTCACGCCATTCT | 19 |
| | | Probe | | |

| Table 2B : SVA targets' primer and probe sequences | | | | |
|---|---|---|---|---|
| Name | Size | Primer Type | Primer & Probe Sequence | SEQ ID NO |
| SVA-100bp | 100bp | Forward | AATGGCGGCTTTGTGGAATA | 20 |
| | | Reverse | GTCTCCCATGTCTACTTCTTTCTAC | 21 |
| | | Probe | AGAAATCGGATGGTTGCCGTGTCT | 22 |
| SVA-101bp | 101bp | Forward | AACCCTGTGCTCTCTGAAAC | 23 |
| | | Reverse | ACGCTGCCTTCAAGCAT | 24 |
| | | Probe | | |
| SVA-103bp | 103bp | Forward | GCCCAACAGCTCATTGAGAA | 25 |
| | | Reverse | ACGGCAACCATCCGATTT | 26 |
| | | Probe | | |
| SVA-104bp | 104bp | Forward | TGTCCACTCAGGGTTAAATGG | 27 |
| | | Reverse | GATTAGGGATTGGTGATAACTCTTA | 28 |
| | | Probe | AAGGGCGGTGCAAGATGTGCTTTGTT | 29 |
| SVA-106bp | 106bp | Forward | TGTGTCCACTCAGGGTTAAAT | 30 |
| | | Reverse | GATTAGGGATTGGTGATGACTCT | 31 |
| | | Probe | AAGGGCGGTGCAAGATGTGCTTTGTT | 32 |
| SVA-106bp-v2 | 106bp | Forward | TGTGCCCAACAGCTCATT | 33 |
| | | Reverse | ACGGCAACCATCCGATTT | 34 |
| | | Probe | | |
| SVA-116bp | 116bp | Forward | CTGTGTCCACTCAGGGTTAAATG | 35 |
| | | Reverse | ATTACTTGAGATTAGGGATTGGTGATG | 36 |
| | | Probe | AAGGGCGGTGCAAGATGTGCTTTGTT | 37 |
| SVA-116bp-v2 | 116bp | Forward | CCCAACAGCTCATTGAGAACG | 38 |
| | | Reverse | CTTTCTACACAGACACGGCAA | 39 |
| | | Probe | | |
| SVA-118bp | 118bp | Forward | CTCTCTGAAACATGTGCTGTGT | 40 |
| | | Reverse | GGGATTGGTGATGACTCTTAACG | 41 |
| | | Probe | AAGGGCGGTGCAAGATGTGCTTTGTT | 42 |
| SVA-118bp-v2 | 118bp | Forward | CTGTGTCCACTCAGGGTTAAAT | 43 |
| | | Reverse | TGATTACTTGAGATTAGGGATTGGT | 44 |
| | | Probe | AAGGGCGGTGCAAGATGTGCTTTGTT | 45 |
| SVA-126bp | 126bp | Forward | CTGTGTCCACTCAGGGTTAAAT | 46 |
| | | Reverse | TGTGTCCCTGATTACTTGAGATTAG | 47 |
| | | Probe | | |
| SVA-126bp-V2 | 126bp | Forward | CCTGTTGATCTGTGACCTTACC | 48 |
| | | Reverse | ACGCTGCCTTCAAGCAT | 49 |
| | | Probe | AAGGGCGGTGCAAGATGTGCTTTGTT | 50 |
| SVA-128bp | 128bp | Forward | GTTGCCGTGTCTGTGTAGAA | 51 |
| | | Reverse | TTTCAGAGAGCACAGGGTTG | 52 |
| | | Probe | AAGGGCGGTGCAAGATGTGCTTTGTT | 53 |
| SVA-132bp | 132bp | Forward | AACCCTGTGCTCTCTGAAAC | 54 |
| | | Reverse | GATTAGGGATTGGTGATAACTCTTA | 55 |
| | | Probe | AAGGGCGGTGCAAGATGTGCTTTGTT | 56 |
| SVA-207bp | 207bp | Forward | CTGTGTCCACTCAGGGTTAAAT | 57 |
| | | Reverse | GAGGGAAGGTCAGCAGATAAAC | 58 |
| | | Probe | AAGGGCGGTGCAAGATGTGCTTTGTT | 59 |
| SVA-257bp | 257bp | Forward | CCTGTGCTCTCTGAAACATGTGCT | 60 |
| | | Reverse | GATTTGGCAGGGTCATGGGACAAT | 61 |
| | | Probe | AAGGGCGGTGCAAGATGTGCTTTGTT | 62 |
| SVA-265bp | 265bp | Forward | ATGTGCTGTGTCCACTCAGGGTTA | 63 |
| | | Reverse | ATTCTTGGGTGTTTCTCACAGAGG | 64 |
| | | Probe | AAGGGCGGTGCAAGATGTGCTTTGTT | 65 |
| SVA-290bp | 290bp | Forward | TGGGATCCTGTTGATCTGTGACCT | 66 |
| | | Reverse | GATTTGGCAGGGTCATGGGACAAT | 67 |
| | | Probe | | |
| SVA-355bp | 355bp | Forward | GTTGCCGTGTCTGTGTAGAA. | 68 |
| | | Reverse | ATGGGACAATAGTGGAGGGA | 69 |
| | | Probe | | |
| SVA-367bp | 367bp | Forward | CCGTGTCTGTGTAGAAAGAAGTAG | 70 |
| | | Reverse | GGGATTTGGCAGGGTCAT | 71 |
| | | Probe | | |
| SVA-399bp | 399bp | Forward | GGCGGCTTTGTGGAATAGA | 72 |
| | | Reverse | GAGGGAAGGTCAGCAGATAAAC | 73 |
| | | Probe | ATCAGGGACACAAACACTGCGGAA | 74 |
| SVA-411bp | 411bp | Forward | TGGAATAGAAAGGCAGGAAAGG | 75 |
| | | Reverse | GCAGGGTCATGGGACAATAG | 76 |
| | | Probe | | |

| Table 2C : Line1 targets' primer and probe sequences | | | | |
|---|---|---|---|---|
| Name | Size | Primer Type | Primer & Probe Sequence | SEQ ID NO |
| Line1-252bp | 252bp | Forward | CACAATAGCAAAGACTTGGAACC | 77 |
| | | Reverse | CCCTTCCTGTGTCCATGTG | 78 |
| | | Probe | CCTTTGTAGGGACATGGATGAAAGTGGA | 79 |
| Line1-257bp | 257bp | Forward | GACTTGGAACCAACCCAAATG | 80 |
| | | Reverse | CCCAGAGTGTGACGTTCC | 81 |
| | | Probe | AGTGAGAACACATGGACACAGGAAGG | 82 |
| Line1-262bp | 262bp | Forward | GTGGCACATATACACCATGGAA | 83 |
| | | Reverse | CGTTAGGTATATCTCCCAATGCTATC | 84 |
| | | Probe | TGAGAACACATGGACACAGGAAGGG | 85 |
| Line1-266bp | 266bp | Forward | ACTTGGAACCAACCCAAATG | 86 |
| | | Reverse | CACAACAGTCCCCAGAGTG | 87 |
| | | Probe | TGAGAACACATGGACACAGGAAGGG | 88 |
| Line1-267bp | 267bp | Forward | CATGGAATACTATGCAGCCATAAA | 89 |
| | | Reverse | CCCACTAACTCGTCATCTAGC | 90 |
| | | Probe | TGAGAACACATGGACACAGGAAGGG | 91 |

The samples used in the methods of this invention may be from a patient has been diagnosed as having a has stage I, stage II, stage III or stage IV cancer, is suffering from cancer, is in remission from cancer, is at risk for developing cancer, has had surgery to remove a tumor, has undergone a neoadjuvant therapy, a targeted therapy, a chemotherapy, immunotherapy and/or radiotherapy to treat a cancer.

The methods of this invention are also useful in further evaluating the patient having a minimum residual disease diagnosis to implement a disease treatment. For example, in an embodiment of this invention a determination is made that the quantity of the short RE targets as compared to the long Re targets is higher in the sample from the patient than that of a control sample, e.g., a sample from a healthy subject, and in view of that determination an appropriate treatment of the patient is instituted, e.g., a targeted therapy, cancer chemotherapy, immunotherapy, or radiotherapy is administered. Such treatment might include e.g., antineoplastic agents, alkylating agents, topoisomerase inhibitors, mitotic inhibitors, methotrexate, vinca alkaloids, antimetabolites, antifolates, pyrimidine antagonists, purine analogs, purine antagonists, proteasome inhibitors, tyrosine kinase inhibitors, nitrogen mustards, or another cancer therapy. Alternatively, a determination of a threshold cycle number of the quantitated nucleic acid fragment, is made and based on that number the clinical provider administers the treatment to the patient.

### Multiplex methods

It is disclosed a method to quantitate the integrity of circulating cell free human DNA and optionally to implement a treatment of a subject, comprising: providing a sample from a subject, preferably a sample that has been treated to remove cells, the sample comprising cell free human DNA comprising a first RE target being 97 base pairs and the second RE target having a length between 260 and 265 base pairs, e.g., 263 bp; using a quantitative polymerase chain reaction (qPCR) method to quantitate the first and second RE targets; obtaining for the quantitated RE targets a threshold cycle number; comparing the threshold cycle number with a standard curve to determine a quantity of each of the RE targets that was present in the sample; calculating a ratio of the quantity of the 97 RE target to the quantity of the between 260 and 265 base pair nucleic acid fragment; and using the quantitated nucleic acid fragment to quantitate the integrity of the circulating cell free human DNA and optionally to implement treatment of a patient. The subj ect's sample may be serum, plasma, urine, or other biological fluid from a human, preferably the sample is a plasma sample. The targets may be amplified in singleplex qPCR wherein a single target is amplified in a single reaction well or the targets may be amplified in a multiplex qPCR wherein all the targets are amplified in a single reaction well.

It is further disclosed a method to quantitate the integrity of circulating cell free human DNA and optionally to implement a treatment of a subject, comprising: providing a sample from a subject, preferably a sample that has been treated to remove cells, , the sample comprising cell free human DNA comprising a first short RE nucleic acid target having a length between 60 and 135 base pairs, 70bp and about 130bp, e.g., 71 and 132 base pairs, or between 60 and 120bp (the first RE target), and the second RE nucleic acid target having a length between 200 to 300 base pairs, between about 207 and 270 bp, or between 260 and 265 base pairs; using a quantitative polymerase chain reaction (qPCR) method to quantitate the first and second RE targets; obtaining for the quantitated RE nucleic acid targets a threshold cycle number; comparing the threshold cycle number with a standard curve to determine a quantity of each of the RE nucleic acid targets that was present in the sample; calculating a ratio of the quantity of the short RE target to the quantity of second RE target; and using the quantitated nucleic acid targets to quantitate the integrity of the circulating cell free human DNA and to implement treatment of a patient. The subject's sample may be serum, plasma, urine, or other biological fluid from a human, preferably the sample is a plasma sample. The first and second RE target may be a target of the same retrotransposable element or may be different retrotransposable elements. If they are from the same retrotransposable element then PCR blockers may be included to limit extension from the primers beyond the position of the blockers, thus limiting the extension from a primer pair used to amplify one RE target into the other RE target and thereby enhancing the specificity by limiting the production of extraneous or overlapping products. In an embodiment the first and second RE targets are targets of an ALU, an SVA or a LINE1 target. In an embodiment the first and second RE targets are targets of an ALU or SVA target or a LINE1 target. Preferably the short RE target is an ALU or an SVA target, e.g., a Yb8 ALU target, and the long RE element is an SVA or LINE1 target. In an embodiment the prime pairs used in the qPCR to quantitate the RE targets are selected from the primer pairs of Table 2A and 2B and 2C . The targets may be amplified in singleplex qPCR wherein a single target is amplified in a single reaction well or the targets may be amplified in a multiplex qPCR wherein all the targets are amplified in a single reaction well.

An embodiment of this invention is a method to quantitate the integrity of circulating cell free human DNA and optionally to implement a treatment of a subject, comprising:
providing a sample from a subject, preferably a sample that has been treated to remove cells, the sample comprising cell free human DNA comprising a two short RE targets, i.e., first RE nucleic acid target and a second RE nucleic acid target having a length of having a length between 60 and 135 base pairs, e.g., 71 and 132 base pairs or between 60 and 120bp;
using a quantitative polymerase chain reaction (qPCR) method to quantitate the first and second targets;
obtaining for the targets a threshold cycle number; comparing the threshold cycle number with a standard curve to determine a quantity of each of the targets that was present in the sample;
calculating the difference between the quantity of the first target and the second target; and
using the quantitated targets to implement treatment of a patient. The subject's sample may be serum, plasma, urine, or other biological fluid from a human, preferably the sample is a plasma sample.
The RE targets may be amplified in singleplex qPCR wherein a single target is amplified in a single reaction well or the targets may be amplified in a multiplex qPCR wherein all the targets are amplified in a single reaction well.

The methods of this invention are contemplated to be useful in identifying a subject having progressive disease or MRD. Accordingly, an embodiment of this invention is a method for identifying a subject having progressive cancer or MRD, said method comprising:
(a) providing a first and second sample of serum, plasma, urine, or other biological fluid from a subject wherein the first and second samples are obtained at least one week apart, at least 2 weeks apart, at least 3 weeks apart or at least 4 weeks apart, e.g., 12 to 21 days apart,
   the samples comprising cell free human DNA (cfDNA), the cfDNA comprising a first and second short retrotransposable interspersed element (RE) target sequence having a length of between about 60 base pairs to about 135 base pairs, wherein the first short target is shorter than the second short RE target;
(b) quantitating each of the short RE targets in the first and second samples using a quantitative polymerase chain reaction (qPCR) method;
(c) obtaining for each of the quantitated RE targets in the first and second samples a threshold cycle number;
(d) comparing the threshold cycle number of each quantitated RE target with a standard curve to determine an amount of each of the quantitated RE targets that were present in the samples;
(e) determining the amount of first short RE target less the amount of the second RE target in the first sample (Frag1) and the amount of first short RE target less the amount of the second RE target in the second sample (Frag2) wherein in increase in Frag2 as compared to Frag1 over a threshold level identifies the subject as having progressive disease or MRD.
The targets may be amplified in singleplex PCR wherein a single target is amplified in a single reaction well or the targets may be amplified in a multiplex PCR wherein all the targets are amplified in a single reaction well.

A subject identified as having progressive disease or MRD may be administered a cancer therapy or MRD therapy. The method may further comprise the step of determining the DNA integrity index (DII) of the cfDNA in the sample

In an embodiment of this method, an increase in Frag2 as compared to Frag1 may be determined by subtracting Frag1 from Frag2 to generate a value, FragDiff, that is compared to a threshold value and based on that comparison it is concluded that the ctDNA has increased and identifies the subject as having progressive disease or MRD and an appropriate therapy may be administered.

Neoadjuvant therapies, which include, e.g., chemotherapy, hormone therapy, immunotherapy, radiation therapy, and targeted therapy are delivered to a subject before the main treatment is administered to help reduce the size of a tumor or kill cancer cells that have spread. Neoadjuvant therapies are recommended when a patient with early-stage cancer, stage I, stage II or stage III, undergoes surgery or radiation therapy. The methods of this invention may be applied to a sample of subject having a stage I, stage II, stage III or stage IV cancer wherein the samples are obtained from the subject before and after the neoadjuvant therapy to quantitate the integrity of circulating cell free human DNA and to implement a treatment of a subject. The methods of this invention may also be applied to samples from a subject who has had a therapy for hepatoma, esophageal cancer, rectal cancer, anal cancer, head and neck cancer, colon cancer, colorectal cancer, lung cancer, breast cancer, neu metastatic breast cancer or a blood cancer, e.g., leukemia, and the first sample was taken from the subject before administering the a first cycle of therapy and the second sample was taken from the subject after administering the first therapy cycle, but before the administration of another cycle of therapy, and as such the first and second samples may be obtained from the subject at least 1 week apart, at least 2 weeks apart, at least 3 weeks apart, at least 4 weeks apart, e.g. 12 to 21 days apart. The therapy may be a targeted therapy, a chemotherapy, immunotherapy or radiotherapy. The therapy may be treatment with an antineoplastic agents, alkylating agents, topoisomerase inhibitors, mitotic inhibitors, methotrexate, vinca alkaloids, antimetabolites, antifolates, pyrimidine antagonists, purine analogs, purine antagonists, proteasome inhibitors, tyrosine kinase inhibitors, nitrogen mustards, immunotherapy, or another cancer therapy.

In the methods described herein for identifying the patient as having progressive disease or MRD, The short and long retrotransposable elements may have a copy number in excess of 1000 copies per genome, e.g., the short retrotransposable interspersed element may be an ALU or an SVA and the long RE may be an ALU, SVA or LINE.

The short RE targets may be from about 60 base pairs to about 135 base pairs, or from about 60 base pairs to about 120 base pairs, or from about 70 base pairs to about 130 base pairs, or from about 80 base pairs to about 100 base pairs. The long RE target may be about 200 bp to about 300 bp or about 207 bp to about 270 bp, or about 260 bp to about 265 bp in length.

The forward and reverse primer pairs used to amplify the short and long target sequences in the qPCR may be selected from the following forward and reverse primer pairs of Tables 2A, 2B, or 2C.

The samples used in the methods described herein may be a sample of serum, plasma, urine, or other biological fluid, preferably the sample is a plasma sample.

The method may further comprise a step of adding a synthetic DNA sequence as an internal positive control (IPC) to the samples prior to quantitating each of the short and long RE targets in the first and second samples by qPCR, and then quantitating the IPC and utilizing the quantitative IPC result in the step of comparing the threshold cycle number of each quantitated RE target with a standard curve to improve the accuracy and reliability of the comparing step. For example, the use of the IPC enables a determination of a concentration of cell free DNA in the sample.

It is specifically contemplated that the quantitation of the short and long retrotransposable interspersed elements of each sample by qPCR may be carried out in a single tube or well.

The amplified RE targets may be detected with one or more hybridization probes that hybridize specifically to the RE targets sequences. The probes may comprise an observable label, e.g., a fluorescent label, e.g., FAM, Cy5, Hex, or Cy3. The observable label could be detected using a microfluidic device. In some embodiments of this invention, the amplification products of the qPCR method used in the methods of this invention may be detected and/or quantified using electrical biosensors (see Liu et al. Single-Nucleotide Polymorphism Genotyping Using a Novel Multiplexed Electrochemical Biosensor with Nonfouling Surface. Biosens. Bioelectron. 2013, 42, 516-521).

Also an embodiment of this invention is a system for characterizing cancer or MRD in a patient, the system comprising:
(a) a sample of serum, plasma, urine, or other biological fluid from a patient, the sample comprising cell free DNA, the cell free DNA comprising a first and second short retrotransposable element targets, the short targets having a length of from about 60 bp to about 135 bp, the sample further comprising an added third target, the third target being an internal positive control (IPC) comprising synthetic DNA;
(b) a TaqMan^{®} probe corresponding to each of the two short targets, and the third target, each probe comprising a detectable label that is distinct from the labels incorporated into the other probes;
(c) a forward primer and a reverse primer pair for amplifying each of the short targets, and the third target;
(d) a DNA standard for generating standard curves for the two short targets;
(e) a qPCR system for simultaneously amplifying the short targets, and the third target and for producing a threshold cycle number for each the short targets and the third target; and
(f) a qPCR data analysis system for producing DNA quantitation values for each retrotransposable element target by interpolation using threshold cycle numbers and standard curves and for using the DNA quantitation values to produce an indication of the amount of the two short retrotransposable element target, and determining a difference in the amount of the first short RE target less the amount of the second RE target compared to a threshold value and characterizing the cancer or MRD.
The system may be a singleplex system wherein a single target is amplified in a single reaction well or the system may be a multiplex system where multiple target are amplified in a single reaction well.

In the system of this invention for characterizing cancer or MRD in a patient the patient may be a patient who is suffering from a cancer, e.g., is diagnosed as having a stage 1, stage II or stage III cancer, is in remission from cancer, is at high risk for developing cancer, has been categorized by another method as having a complete response ("CR"), a stable disease ("SD"), a partial response ("PR"), or progressive disease ("PD"), or has had a neoadjuvant therapy, has had surgery to remove a tumor, or has undergone chemotherapy, immunotherapy or radiotherapy to treat the cancer or MRD. The cfDNA in the multiplex system may further comprise cfDNA comprising a long retrotransposable element target having a length of between 200 bp and 300 bp, or 207 bp to 270 bp, e.g., 260-267 bp, a TaqMan probe corresponding to the long RE target, and forward and reverse primers for amplifying the long RE target. In the system, the forward primer and reverse primer pair for amplifying the RE targets are selected from Table 2A, 2B or 2C.

The method of this invention are contemplated for allow for the quantitated RE target amounts to be correlated to one cancer cell in 500,000 total cells or greater, one cancer cell in 1,000,000 total cells or greater, one cancer cell in 1,500,000 cells or greater.

### EXAMPLES

### Example 1 - Protocol for Serum and Plasma Separation

Serum and plasma separation were performed according to the standard protocol and within four hours of collection, and stored at -80°C until they were processed. Care was taken to avoid freeze-thaw cycles. For serum specimens, whole blood is collected in the commercially available red-topped test tube Vacutainer (Becton Dickinson). For plasma specimens, whole blood is collected in the commercially available anticoagulant-treated tubes e.g., EDTA-treated or citrate-treated.

### Example 2 - Protocol for Direct DNA Quantitation

Two separate protocols have previously been described for direct DNA quantification from either human serum (Umetani, N., et al., Increased integrity of free circulating DNA in sera of patients with colorectal or periampullary cancer: Direct quantitative PCR for ALU repeats, Clin. Chem. 52(6): 1062-1069 (2006), doi:10.1373/clinchem.2006.068577, incorporated herein in its entirety) or plasma (Breitbach, S, et al., Direct quantification of cell-free, circulating DNA from unpurified plasma, PLOS One 9(3): e87838 (2014), doi:10.1371/journal.pone.0087838, incorporated herein in its entirety). We used both of these methods on serum and plasma and compared the amplification efficiency from both methods. The first method includes deactivation or elimination of proteins that bind to template DNA or DNA polymerase and might invalidate qPCR results. Briefly, a volume of 20 µL of each serum or plasma sample was mixed with 20 µL of a preparation buffer that contains 25 mL/L Tween 20, 50 mM Tris, and 1 mM EDTA. This mixture was then digested with 16 µg of proteinase K solution (Qiagen) at 50°C. for 20 min, followed by 5 min of heat deactivation and inactivation at 95°C. After subsequent centrifugation at 10,000 g for 5 min, 0.2 µL of the supernatant (containing 0.1-µL equivalent volume of serum or plasma) was used as a template for each direct RE-qPCR reaction. The second method bypasses the protein removal step and only requires 1:40 dilution of the serum/plasma sample with sterile H₂O.

### Example 3 - Procedure for DNA Purification

For comparison to and validation of direct quantification of cfDNA, RE-qPCR has been performed on isolated, purified cfDNA. cfDNA was purified by magnetic bead extraction or by using the silica based membrane QIAamp DNA Investigator Kit (Qiagen).

### Example 4 - Design of Primers and TaqMan^{®} Probes for ALU Yb8 and SVA targets

In general primers and labeled probes used in the qPCR reactions may be obtained from Eurofins MWG/Operon, Integrated DNA Technologies, or a variety of other vendors.

Short ALU primer sets were designed to produce amplicon lengths of 80 bp, 97 bp, 105bp, 120 bp, and 123 bp among others, were developed for use in the assays of the present invention. The primer sequences are shown in Table 2A. Primer pairs to produce amplicon lengths from SVA of 100bp, 104bp, 106 bp, 116bp, 118bp, 126bp, 132bp, 207 bp, 257 bp, 265 bp, 290 bp, or to produce amplicon lengths of 252bp, 257bp, 262bp, and 267bp from LINE1, among others are set forth in Table 2B and 2C. The primer pairs were developed using Primer 3 software and an SVA or LINE1 (genebank ID: AH005269 (PUBMED 10655552) retrotransposon sequence. Because the SVA and LINE1 sequences are truncated in many individuals and also have sequence similarities with ALU sequences in certain regions, the target SVA sequences were selected from the SVA-R region, and the target LINE1 sequence was selected from the LINE1 ORF2 region, which have no or minimal sequence similarity as compared with the ALU sequence. The primer sequences and probes that hybridize to the amplified targets are shown in Table 2A, 2B and 2C.

Additional primer design based on ALU Yb8, SVA and LINE1 may be done using Primer software (Koressaar, T; Remm, M, Bioinformatics 23(10): 1289-91 (2007), doi:10.1093/bioinformatics /btm091; Untergasser A, et al., Nucleic Acids Res. 40(15): e115 (2012), doi:10.1093/nar/gks596).

### Example 5 - Procedure for qPCR

The qPCR assays were run on an Applied Biosystems 7500 Real Time PCR instrument and/or the Biorad CFX, but useful instrument platforms are not limited thereto. The qPCR assays of the present invention may be adapted to work on most Real-Time PCR instruments. To assess the concentration and integrity index of serum and plasma circulating cfDNA, both short and long fragments may be amplified and quantified. The short fragment primer sets may amplify the short (apoptotic) DNA fragments, whereas the long fragment primer sets may amplify the long (non-apoptotic) DNA fragments. The RE-qPCR multiplex reaction may contain three targets in a Taqman based assay: a short RE target, a long RE target, and a synthetic IPC sequence. The hybridization probes detecting each target may be labeled with different fluorophores (e.g. FAM, Cy5, Hex, or Cy3) to enable simultaneous detection. The following PCR conditions may be used, but they can be modified as necessary: 10 min 95°C denaturation cycle, followed by 32 cycles of 2-step qPCR (15 s at 95 °C and 2 min at 61 °C combined annealing/extension time) at maximum ramp speed. Additional PCR parameters (i.e. cycle number, denaturation and annealing/extension times and temperatures) are investigated to obtain a robust, sensitive qPCR multiplex.

Short Yb8 and long SVA primer pairs selected from those shown in Table 2A and 2B were combined into eight different multiplex sets (Yb8-80 & SVA-207, Yb8-80 & SVA-257, Yb8-80 & SVA-265, Yb8-80 & SVA-290, Yb8-120 & SVA-207, Yb8-120 & SVA-257, Yb8-120 & SVA-265, and Yb8-120 & SVA-290). The optimal temperature for each multiplex was determined by a temperature gradient ranging from 64.0 °C to 55.0 °C. The concentration of primers and additives including DMSO and additional MgCh were optimized for each multiplex set.

The reaction mixture of each multiplex Yb8-SVA-qPCR included a template, forward primer and reverse primer pairs, fluorescent probe, Brilliant Multiplex QPCR Master Mix (Agilent) and the additives bovine serum albumin (BSA), dimethyl sulfoxide (DMSO), and magnesium chloride (MgCh). Real-time PCR amplification was performed with pre-cycling heat activation of DNA polymerase at 95 °C for 10 min followed by 32 cycles of denaturation at 95 °C for 15 sec and extension at 61-62.5 °C (adapted to the multiplex set) in a CFX96 Touch Real-Time PCR Detection System (Bio-Rad Laboratories). The quantification of DNA in each sample was determined by use of a calibration curve with serial dilutions (20ng/ul to 0.6pg/ul).

### Example 6 - Procedure for qPCR - 97 bp fragment of ALU Yb8

The qPCR assays may be run on an Applied Biosystems 7500 Real Time PCR instrument and/or the Biorad CFX, but useful instrument platforms are not limited thereto. The qPCR assays of the present invention may be adapted to work on most Real-Time PCR instruments. To assess the concentration of a 97bp target of ALU Y8b in plasma circulating cfDNA in control healthy subjects compared to cancer patients, plasma samples of control (healthy subjects) and test (samples from patients with metastatic colorectal cancer (mCRC)) containing cfDNA was combined with the 97 bp forward primer (GTGGCTCACGCCTGTAAT)(SEQ ID NO: 7) , 97 bp reverse primer (GGGTTTCACCTTGTTAGCCA) (SEQ ID NO: 8), a fluorescent probe comprising TGGATCATGAGGTCAGGAGAT (SEQ ID NO: 9), Brilliant Multiplex QPCR Master Mix (Agilent) and the additives bovine serum albumin (BSA), dimethyl sulfoxide (DMSO), and magnesium chloride (MgCh). Real-time PCR amplification was performed with pre-cycling heat activation of DNA polymerase at 95 °C for 10 min followed by 40 cycles of denaturation at 90-95 °C for 10-15 sec and extension at 61-64 °C (depending on the multiplex set) in an ABI 7500 Instrument (ThermoFisher Scientific). The quantification of DNA in each sample was determined by use of a calibration curve with serial dilutions (20 ng/ul to 0.6 pg/ul)

### Example 7 - Procedure for qPCR 97 bp ALU Yb8 and 80 bp ALU Yb8

To assess the concentration of plasma circulating cfDNA, both 80bp and 97bp fragments of ALU Yb8 are amplified and quantified. The RE-qPCR multiplex reaction contains three targets in a TaqMan^{®} based assay: 80 bp ALU Yb8 forward and reverse primers (GGAAGCGGAGCTTGCAGTGA (SEQ ID NO:1) and AGACGGAGTCTCGCTCTGT CGC (SEQ ID NO: 2)) , the 97 bp ALU Yb8 RE forward and reverse primers GTGGCTCACGCCTGTAAT (SEQ ID NO: 7) and GGGTTTCACCTTGTTAGCCA(SEQ ID NO:8)), an 80R- blocker, peptide nucleic acid (PNA) oligo which binds to the 80 bp ALU Yb8 fragment, a 97R-PNA blocker a PNA which binds to the 97 bp ALU Yb8 fragment and probes that hybridize to the 80 bp and the 97 bp sequences (e.g., Fluorophore-TGAGGTCAGGAGATCGAGACCATCC-Quencher)(SEQ ID NO: 92), and in some instances a synthetic IPC sequence. PNA oligo mimics DNA. In PNA, the negatively-charged sugar phosphate backbone of DNA is replaced with an uncharged pseudo-peptide backbone. The two strands of a PNA/DNA hybrid therefore lack the electrostatic repulsion as observed for DNA/DNA duplexes, giving rise to thermal stability. Hybridization probes are also included in some instances for detecting each target and the probes are labeled with different fluorophores (e.g. FAM, Cy5, Hex, or Cy3) to enable simultaneous detection. The reaction mixture includes the forward primers, reverse primers, the blockers, the fluorescent probe, Brilliant Multiplex QPCR Master Mix (Agilent) and the additives bovine serum albumin (BSA), dimethyl sulfoxide (DMSO), and magnesium chloride (MgCh). Real-time PCR amplification is performed with pre-cycling heat activation of DNA polymerase at 95 °C for 10 min followed by 32 cycles of denaturation at 95 °C for 15 sec and extension at 61-62.5 °C (depending on the multiplex set) in a CFX96 Touch Real-Time PCR Detection System (Bio-Rad Laboratories). The quantification of DNA in each sample is determined by use of a calibration curve with serial dilutions (20ng/ul to 0.6pg/ul).

Figure 2 shows the two PCR target regions of 80bp and 97bp on the Yb8 sequence. In order to amplify the 97bp and 80bp separately, we use two peptide nucleic acid (PNA) oligos to block PCR extension beyond the target regions. PNA oligos are used as sequence specific PCR blockers because PNA probes have strong binding affinity and specificity to its target DNA and are not recognized by DNA polymerase as primer. In the diagram 97F-Blocker binds the complement sequence of Alu-Yb8 between 97bp and 80bp target regions and prevents DNA elongation from the 97bp forward primer beyond the region where the 97bp reverse primer binds. In a similar way, 80R-Blocker binds Alu-Yb8 sequence between 97bp and 80bp target regions and prevents DNA elongation from the 80bp reverse primer beyond the region where the 80bp forward primer binds. By incorporating these two PCR blockers, it is possible to prevent PCR amplification of nearly entire Alu-Yb8 sequence that can occur with the 97F/80R primer pair. We are then able to compare the amounts of the 80 bp and 97 bp fragments in the plasma of control samples from healthy subjects and the plasma from cancer patients.

### Example 8 - Procedure for qPCR 80 bp ALU Yb8 and 120 bp ALU Yb8 and 265 SVA

Plasma from 40 control subjects, healthy subjects without cancer and 39 subjects having cancer were subjected to qPCR assays to assess the level of ctDNA. The qPCR assays were run on an Applied Biosystems 7500 Real Time PCR instrument and/or the Biorad CFX, but useful instrument platforms are not limited thereto. The qPCR assays of the present invention may be adapted to work on most Real-Time PCR instruments. To assess the concentration and integrity index of serum circulating cfDNA in the samples from control and cancer subject, a first ALU Yb8 target of 80 bp, and a second ALU Yb8 target of 120 bp and an SVA target of 265 bp were amplified and quantified in a RE-qPCR multiplex reaction. The RE-qPCR multiplex reaction contained three targets in a Taqman based assay: the first ALU Yb8 target of 80 bp , the second ALU Yb8 target of 120 bp, a third SVA target of 265 bp, and a synthetic internal positive control (IPC) sequence. The hybridization probes detecting each amplified target were labeled with different fluorophores (FAM, Cy5, or Hex) to enable simultaneous detection. The following PCR conditions are used: 10 min 95°C denaturation cycle, followed by 40 cycles of 2-step qPCR (15 s at 96 °C and 2 min at 64 °C combined annealing/extension time) at maximum ramp speed.

The reaction mixture of each multiplex Yb8-qPCR included the forward primers and reverse primers for the first Yb8-80 target and for the second ALU Yb8-120 target, and the long SVA 265 target (see Table 2A and 3B for primer pair sequences). the fluorescent probes for detecting the amplified fragments, Brilliant Multiplex QPCR Master Mix (Agilent) and the additives bovine serum albumin (BSA), dimethyl sulfoxide (DMSO), and magnesium chloride (MgCl₂). Real-time PCR amplification was performed with pre-cycling heat activation of DNA polymerase at 95 °C for 10 min followed by 40 cycles of denaturation at 95 °C for 15 sec and extension at 61-62.5 °C (depending on the multiplex set) in a CFX96 Touch Real-Time PCR Detection System (Bio-Rad Laboratories). The quantification of the targets in each sample was determined by use of a calibration curve with serial dilutions (20ng/ul to 0.6pg/ul). Table 3 sets forth the quantitated amounts of the 80bp target, the 120 bp target and the 265 bp target in each sample and the difference in the quantitated amount of 80 bp Yb8 target and the quantitated amount of 120 bp Yb8 target.

| **Table 3** | | | | | | | | | |
|---|---|---|---|---|---|---|---|---|---|
| **Healthy Control (ng/mL)** | | | | | **Cancer patient (ng/mL)** | | | | |
| | | | | **80bp-120bp** | | | | | **80bp-120bp** |
| Median | | | | 1.85 | Median | | | | 25.60 |
| Average | | | | 2.22 | Average | | | | 217.52 |
| SD | | | | 1.87 | SD | | | | 793.35 |

| | Target Size (bp) | | | | | Target Size (bp) | | | |
|---|---|---|---|---|---|---|---|---|---|
| Sample ID# | 80bp | 120bp | 265bp | **80bp-120bp** | Sample ID# | 80bp | 120bp | 265bp | **80bp-120bp** |
| 1287 | 4.68 | 2.98 | 1.06 | 1.69 | 1683 | 16.13 | 10.07 | 3.19 | 6.06 |
| 1289 | 4.77 | 3.24 | 1.01 | 1.53 | 1685 | 1125.52 | 468.29 | 114.47 | 657.23 |
| 1291 | 5.51 | 3.64 | 0.79 | 1.88 | 1689 | 24.00 | 14.81 | 3.54 | 9.19 |
| 1293 | 9.93 | 6.13 | 2.04 | 3.80 | 1691 | 108.65 | 50.63 | 8.78 | 58.02 |
| 1295 | 20.82 | 9.56 | 2.25 | 11.26 | 1693 | 30.47 | 17.16 | 3.94 | 13.31 |
| 1299 | 11.79 | 6.52 | 1.99 | 5.26 | 1695 | 109.66 | 61.52 | 12.89 | 48.14 |
| 1301 | 7.40 | 5.32 | 2.02 | 2.08 | 1697 | 142.73 | 71.97 | 16.07 | 70.76 |
| 1303 | 12.19 | 5.93 | 2.37 | 6.26 | 1699 | 98.21 | 46.29 | 10.31 | 51.92 |
| 1305 | 5.07 | 3.73 | 1.26 | 1.34 | 1701 | 458.87 | 163.28 | 36.89 | 295.60 |
| 1307 | 3.71 | 2.17 | 0.96 | 1.53 | 1703 | 19.19 | 8.19 | 2.30 | 11.00 |
| 1311 | 8.89 | 4.87 | 1.40 | 4.02 | 1705 | 36.65 | 17.82 | 6.06 | 18.84 |
| 1313 | 5.47 | 3.03 | 0.88 | 2.44 | 1707 | 25.60 | 12.62 | 2.13 | 12.98 |
| 1315 | 4.60 | 2.59 | 1.04 | 2.01 | 1709 | 14.10 | 8.33 | 2.83 | 5.77 |
| 1316 | 3.07 | 1.72 | 0.59 | 1.35 | 1711 | 66.40 | 30.19 | 8.93 | 36.21 |
| 1317 | 4.17 | 2.19 | 0.73 | 1.98 | 1713 | 116.21 | 67.75 | 16.99 | 48.47 |
| 1318 | 5.92 | 3.09 | 1.18 | 2.84 | 1715 | 86.71 | 39.03 | 3.47 | 47.68 |
| 1319 | 4.15 | 2.98 | 1.38 | 1.17 | 1717 | 154.01 | 56.55 | 16.67 | 97.46 |
| 1320 | 6.82 | 4.77 | 1.73 | 2.05 | 1719 | 69.53 | 26.53 | 6.86 | 43.00 |
| 1321 | 3.09 | 1.93 | 0.65 | 1.16 | 1721 | 22.51 | 11.52 | 3.22 | 10.98 |
| 1322 | 2.25 | 1.61 | 0.64 | 0.65 | 1723 | 7395.87 | 2497.71 | 372.11 | 4898.16 |
| 1323 | 3.97 | 2.09 | 0.81 | 1.88 | 1725 | 182.55 | 95.65 | 32.66 | 86.91 |
| 1324 | 5.49 | 3.63 | 1.55 | 1.86 | 1727 | 10.26 | 6.80 | 2.77 | 3.46 |
| 1325 | 4.74 | 3.13 | 0.90 | 1.61 | 1729 | 281.95 | 104.02 | 31.17 | 177.93 |
| 1329 | 2.27 | 1.69 | 0.63 | 0.58 | 1731 | 401.77 | 168.31 | 39.10 | 233.46 |
| 1331 | 3.29 | 1.99 | 0.34 | 1.31 | 1733 | 17.73 | 8.26 | 2.78 | 9.47 |
| 1333 | 8.64 | 6.80 | 1.94 | 1.84 | 1735 | 21.73 | 11.47 | 2.26 | 10.26 |
| 1421 | 6.73 | 4.30 | 1.72 | 2.43 | 1737 | 67.34 | 29.16 | 8.38 | 38.18 |
| 1431 | 10.39 | 8.24 | 2.77 | 2.15 | 1739 | 55.40 | 29.80 | 8.20 | 25.60 |
| 1435 | 5.00 | 3.93 | 1.24 | 1.07 | 1741 | 381.36 | 178.68 | 46.72 | 202.68 |
| 1439 | 7.39 | 4.24 | 1.13 | 3.15 | 1743 | 38.51 | 15.86 | 3.56 | 22.66 |
| 1441 | 2.56 | 1.62 | 0.54 | 0.94 | 1745 | 1643.19 | 612.70 | 129.68 | 1030.49 |
| 1443 | 6.03 | 4.12 | 1.30 | 1.91 | 1747 | 18.35 | 8.52 | 2.51 | 9.83 |
| 1447 | 2.51 | 1.97 | 0.67 | 0.54 | 1749 | 23.30 | 14.88 | 5.17 | 8.43 |
| 1449 | 4.52 | 2.80 | 1.15 | 1.72 | 1751 | 53.04 | 46.31 | 31.81 | 6.74 |
| 1451 | 10.17 | 8.20 | 2.65 | 1.98 | 1753 | 31.03 | 17.16 | 4.19 | 13.87 |
| 1453 | 4.73 | 3.49 | 1.09 | 1.24 | 1755 | 22.51 | 13.15 | 4.35 | 9.37 |
| 1455 | 7.34 | 5.81 | 1.63 | 1.54 | 1757 | 8.00 | 5.37 | 1.85 | 2.64 |
| 1457 | 7.43 | 6.18 | 1.23 | 1.25 | 1759 | 46.28 | 36.00 | 8.47 | 10.28 |
| 1459 | 7.61 | 5.60 | 1.90 | 2.00 | 1761 | 282.85 | 142.45 | 25.42 | 140.40 |
| 1765 | 4.77 | 3.36 | 1.16 | 1.41 | | | | | |

### Example 9 - Procedure for qPCR Data Analysis and Quality Control

Data analysis was performed utilizing the respective AB 7500, QuantStudio-5 or BioRad CFX instrument software. Melt curve analysis was generated using Qiagen's QuantiTect 1 SYBR1 Green PCR Kit (Cat#204141) and operated using the Applied Biosystems 7500 Real Time PCR instrument. For each experiment, a freshly prepared 3-fold serial dilution of high molecular weight standard DNA (ranging from 10 ng/µL to 0.004 ng/µL) was run in duplicate on each plate to generate standard curves for the long and short targets. The standard curves are plotted C_{T} vs. Delta Rₙ (the fluorescence emission intensity of the reporter dye divided by the fluorescence emission intensity of the passive reference dye). Resultant DNA quantitation values are interpolated from the resulting linear standard curves. At least one negative No Template Control (NTC) was run on each plate.

In experiments where a ratio between DNA concentration of a 265bp SVA long target and 80bp ALU short target were calculated, the DNA concentration of the long target divided by DNA concentration of the short target provides an indication as to the degree of DNA integrity for the quantified sample. DNA integrity index is calculated as the ratio of concentrations ([concentration of long RE marker]/[concentration of short RE marker]). Quality metrics, including PCR efficiencies (i.e. slope) of both short and long targets, Y-intercept values, and verification of no true amplification in negative controls was assessed.

### Example 10 - Identifying Patients with Increasing ctDNA

Blinded samples of plasma from 66 cancer patients were subjected to a qPCR to assess the level of ctDNA using the methods described herein and identify patients as having progressive disease. The plasma samples were from patients who had been previously diagnosed as having either colorectal cancer, non-small cell lung cancer, small cell lung cancer or breast cancer and had received either chemotherapy, targeted therapy, immunotherapy, or a combination of therapies.

A first plasma sample was obtained from the patients before receiving a cycle of therapy and a second plasma sample was obtained 12 days to 21 days after the cycle of therapy and before receiving another cycle of therapy. The qPCR assays were run on an Applied Biosystems 7500 Real Time PCR instrument and/or the Biorad CFX, but useful instrument platforms are not limited thereto and the qPCR assays of the present invention may be adapted to work on most Real-Time PCR instruments.

To assess the concentration and integrity index of the cfDNA in the samples from control and cancer patients, a first ALU Yb8 target of 80 bp, and a second ALU Yb8 target of 105 bp and an SVA target of 265 bp were amplified and quantified in a RE-qPCR multiplex reaction. The sequence of the primer pairs used to amplify yb-8-80, yb-8 105 and SVA 265 are set forth in Table 1. The RE-qPCR multiplex reaction was a Taqman^{®} based assay comprising Brilliant Multiplex QPCR Master Mix (Agilent), bovine serum albumin (BSA), dimethyl sulfoxide (DMSO), and magnesium chloride (MgCh), and comprised the plasma sample, a primer pair for amplifying the first ALU yb-8 target of 80 bp, a primer pair for amplifying the second ALU Yb8 target of 105 bp, and a primer pair for amplifying a third SVA target of 265 bp, and a synthetic internal positive control (IPC). The amplification products were detected with hybridization probes for the Yb-8 80bp target, the Yb8-105bp target and the SVA 265 bp target, each labeled with a different fluorophore to enable simultaneous detection of the different amplified targets.

Real-time PCR amplification was performed with pre-cycling heat activation of DNA polymerase in a QuantStudio-5 (Thermofisher Scientifics). The quantification of the RE targets in each sample was determined by use of a calibration curve with serial dilutions (20ng/ul to 0.6pg/ul) and the difference between the amount of the first Yb8 80bp target and the amount of the second Yb8 105bp target in the first sample (Frag1) and the amount of the first Yb8 80bp target and the amount of the second Yb8 105bp target in the second samples of plasma (Frag2) were calculated. The difference between Frag2 and Frag1 (Frag Diff) was also calculated. An increase in the amount of the 80bp yb-8 target as compared to the 105bp yb-8 target in the second sample as compared to the first sample indicated an increase in the ctDNA.

Figure 3 depicts the FragDiff of the 66 patients, who had been classified as having progressive disease (triangles), or having non-progressive disease (circles). Figure 3 demonstrates that the method disclosed herein rapidly assesses cfDNA integrity. Figure 3 also demonstrates that based upon the FragDiff being above a threshold level, the method rapidly and reliably identifies a patient as having progressive disease see also Figure 4. Thus, the method described herein can also be used as a factor for rapidly concluding the patient has progressive disease or the cancer treatment was not effective. This is in contrast to other standard assays, e.g., CT scans, Xrays, and CEA measurements, that require weeks, if not months, before it is determined the patient has progressive disease and a therapy can be identified as ineffective.

While the invention has been described and illustrated with reference to certain particular embodiments thereof, those skilled in the art will appreciate that various adaptations, changes, modifications, substitutions, deletions, or additions of procedures and protocols may be made. It is intended, therefore, that the invention be defined by the scope of the claims that follow and that such claims be interpreted as broadly as is reasonable.

## Claims

1. A method to quantitate the integrity of circulating cell free human DNA and implement a treatment of a patient, said method comprising:
(a) providing a first and second sample of serum, plasma, urine, or other biological fluid from a subject wherein the first and second samples are obtained at least one week apart, at least 2 weeks apart, at least 3 weeks apart or at least 4 weeks apart, the first and second samples comprising cell free human DNA (cfDNA), the cfDNA comprising a first and second short retrotransposable interspersed element (RE) target sequence having a length of between about 60 base pairs to about 135 base pairs, wherein the first and second short targets differ in length;
(b) quantitating each of the first and second short RE targets in the first and second samples using a quantitative polymerase chain reaction (qPCR) method;
(c) obtaining for each of the quantitated RE targets in the first and second samples a threshold cycle number;
(d) comparing the threshold cycle number of each quantitated RE target with a standard curve to determine an amount of each of the quantitated RE targets that were present in the samples, wherein the amount of short RE targets in the second samples is indicative of the integrity of the circulating cell free DNA;
(e) determining an increase in the amount of short RE targets in the second sample as compared to the first sample above a threshold level, and
(f) determining a treatment for a patient having an increase in the amount of short RE targets in the second sample as compared to the first sample above a threshold level.

2. The method of claim 1 wherein the first sample is obtained from the subject prior to administration of a neoadjuvant and the second sample is obtained from the subject after the neoadjuvant is administered and before another therapy is administered; or wherein the first sample is obtained from the subject prior to administration of a cycle of therapy and the second sample is obtained from the subject after the cycle of therapy is administered and before a next cycle of therapy is administered.

3. The method of claim 1, wherein steps (a) through (f) are repeated through multiple cycles of therapy.

4. The method of claim 3, wherein an increase in the amount of short RE targets in the second sample as compared to the first sample above a threshold level identifies the therapy as ineffective.

5. The method of claim 1, wherein an increase in the amount of short RE targets in the second sample as compared to the first sample above a threshold level identifies the patient as having progressive disease, or wherein an increase in the amount of short RE targets in the second sample as compared to the first sample above a threshold level identifies the patient as having minimum residual disease (MRD).

6. The method of claim 1 wherein the short RE targets have a length from 70 bp to about 130 bp, or from 60 bp to 120 bp; preferably the first short RE targets has a length between 70 and 80bp, and the second short RE target has a length between 105 and 120bp.

7. The method of claim 1 wherein the qPCR method uses primer pairs set forth in Table 2A or 2B.

8. The method of claim 1, wherein the forward primer and reverse primer pair for amplifying the short target sequences are selected from the following forward and reverse primer pairs,
| Fragment name | Size | Primer Type | Primer Sequence | SEQ ID NO |
|---|---|---|---|---|
| Yb8-80bp | 80bp | Forward | GGAAGCGGAGCTTGCAGTGA | |
| | | Reverse | AGACGGAGTCTCGCTCTGTCGC | |
| Yb8-71bp | 71bp | Forward | CTTGCAGTGAGCCGAGATT | |
| | | Reverse | GAGACGGAGTCTCGCTCTGTC | |
| Yb8-97bp | 97bp | Forward | GTGGCTCACGCCTGTAAT | |
| | | Reverse | GTGGCTCACGCCTGTAAT | |
| Yb8-105bp | 105bp | Forward | AGGCAGGAGAATGGCGTGAACC | |
| | | Reverse | AGACGGAGTCTCGCTCTGTCGC | |
| Yb8-120bp | 120bp | Forward | TGGATCATGAGGTCAGGAGAT | |
| | | Reverse | CCGAGTAGCTGGGACTACA | |
| SVA-100bp | 100bp | Forward | AATGGCGGCTTTGTGGAATA | |
| | | Reverse | GTCTCCCATGTCTACTTCTTTCTAC | |
| SVA-101bp | 101bp | Forward | AACCCTGTGCTCTCTGAAAC | |
| | | Reverse | ACGCTGCCTTCAAGCAT | |
| SVA-103bp | 103bp | Forward | GCCCAACAGCTCATTGAGAA | |
| | | Reverse | ACGGCAACCATCCGATTT | |
| SVA-104bp | 104bp | Forward | TGTCCACTCAGGGTTAAATGG | |
| | | Reverse | GATTAGGGATTGGTGATAACTCTTA | |
| SVA-106bp | 106bp | Forward | TGTGTCCACTCAGGGTTAAAT | |
| | | Reverse | GATTAGGGATTGGTGATGACTCT | |
| SVA-106bp-v2 | 106bp | Forward | TGTGCCCAACAGCTCATT | |
| | | Reverse | ACGGCAACCATCCGATTT | |
| SVA-116bp | 116bp | Forward | CTGTGTCCACTCAGGGTTAAATG | |
| | | Reverse | ATTACTTGAGATTAGGGATTGGTGATG | |
| SVA-116bp-v2 | 116bp | Forward | CCCAACAGCTCATTGAGAACG | |
| | | Reverse | CTTTCTACACAGACACGGCAA | |
| SVA-118bp | 118bp | Forward | CTCTCTGAAACATGTGCTGTGT | |
| | | Reverse | GGGATTGGTGATGACTCTTAACG | |
| SVA-118bp-v2 | 118bp | Forward | CTGTGTCCACTCAGGGTTAAAT | |
| | | Reverse | TGATTACTTGAGATTAGGGATTGGT | |
| SVA-126bp | 126bp | Forward | CTGTGTCCACTCAGGGTTAAAT | |
| | | Reverse | TGTGTCCCTGATTACTTGAGATTAG | |
| SVA-126bp-V2 | 126bp | Forward | CCTGTTGATCTGTGACCTTACC | |
| | | Reverse | ACGCTGCCTTCAAGCAT | |
| SVA-128bp | 128bp | Forward | GTTGCCGTGTCTGTGTAGAA | |
| | | Reverse | TTTCAGAGAGCACAGGGTTG | |
| SVA-132bp | 132bp | Forward | AACCCTGTGCTCTCTGAAAC | |
| | | Reverse | GATTAGGGATTGGTGATAACTCTTA | |

9. The method of claim 1, further comprising a step of adding a synthetic DNA sequence as an internal positive control prior to step (b), quantitating the internal positive control in step (b), and utilizing the quantitative internal positive control result in the comparing step to improve the accuracy and reliability of the comparing step; preferably the use of the internal positive control enabling a determination of a concentration of cell free DNA in the sample.

10. The method of claim 1, steps (b)-(f) are being carried out in a single tube or well.

11. The method of claim 1, the providing step further comprising providing a hybridization probe that hybridizes to the RE target; preferably the hybridization probe including an observable label; more preferably the observable label being a fluorescent label.

12. The method of claim 1, wherein the amount of the first and second short RE targets in the first and second samples amplified in the quantitative polymerase chain reaction (qPCR) method are measured using an electrical biosensor.

13. The method of claim 1, wherein the quantitated short RE target amount represents one cancer cell in 500,000 total cells or greater, 1,000,000 total cells or greater or 1,500,000 cells or greater in the patient.

14. A multiplexed system for carrying out a method according to one of claims 1 to 13 for identifying an ineffective neoadjuvant or cancer treatment, or for characterizing cancer or MRD in humans, the system comprising:
a. a sample of serum, plasma, urine, or other biological fluid from a human, the sample comprising cell free DNA, the cell free DNA comprising two short RE targets, the short RE targets having a length between 60 bp and 135 bp with the proviso that the two short RE targets differ in size sufficiently to distinguish their amplicons, the sample further comprising an internal positive control (IPC) comprising synthetic DNA;
b. a TaqMan^{®} probe corresponding to each of the short RE targets and the IPC, each probe comprising a detectable label that is distinct from the labels incorporated into the other probes;
c. a forward primer and a reverse primer for amplifying each of the short RE target and the IPC;
d. a DNA standard for generating standard curves for RE targets;
e. a qPCR system for simultaneously amplifying the short RE targets and the IPC and for producing a threshold cycle number for each target; and
f. a qPCR data analysis system for producing DNA quantitation values for each RE target by interpolation using threshold cycle numbers and standard curves and for using the DNA quantitation values to produce an indication of the integrity of the cell free DNA.

15. The system of claim 14, wherein the two short RE targets differ in length by at least 10 bp, 15 bp, 20 bp, or 25 bp.

## Patentansprüche

1. Verfahren zur Quantifizierung der Integrität zirkulierender zellfreier humaner DNA und Implementierung einer Behandlung eines Patienten, wobei das Verfahren Folgendes umfasst:
(a) Bereitstellen einer ersten und einer zweiten Probe von Serum, Plasma, Urin oder einer anderen biologischen Flüssigkeit von einer Person, wobei die erste und die zweite Probe in einem Mindestabstand von einer Woche, in einem Mindestabstand von 2 Wochen, in einem Mindestabstand von 3 Wochen oder in einem Mindestabstand von 4 Wochen gewonnen werden, die erste und die zweite Probe zellfreie humane DNA, *cell free human DNA* - (cfDNA), umfassen, die cfDNA eine erste und eine zweite kurze Zielsequenz mit retrotransponierbaren eingestreuten Elementen, *retrotransposable interspersed element* - (RE), mit einer Länge zwischen ungefähr 60 Basenpaaren und ungefähr 135 Basenpaaren umfasst, wobei das erste und das zweite kurze Ziel unterschiedlich lang sind;
(b) Quantifizieren jedes des ersten und des zweiten kurzen RE-Ziels in der ersten und der zweiten Probe unter Verwendung eines Verfahrens der quantitativen Polymerase-Kettenreaktion, *quantitative polymerase chain reaction* - (qPCR);
(c) Erlangen, für jedes der quantifizierten RE-Ziele in der ersten und der zweiten Probe, einer Schwellenzyklusanzahl;
(d) Vergleichen der Schwellenzyklusanzahl jedes quantifizierten RE-Ziels mit einer Standardkurve, um eine Menge jedes der quantifizierten RE-Ziele, die in den Proben vorhanden waren, zu bestimmen, wobei die Menge von kurzen RE-Zielen in den zweiten Proben die Integrität der zirkulierenden zellfreien DNA angibt;
(e) Bestimmen einer Zunahme der Menge von kurzen RE-Zielen in der zweiten Probe im Vergleich zu der ersten Probe oberhalb eines Schwellenwertes und
(f) Bestimmen einer Behandlung für einen Patienten, der eine Zunahme der Menge von kurzen RE-Zielen in der zweiten Probe im Vergleich zu der ersten Probe oberhalb eines Schwellenwertes aufweist.

2. Verfahren nach Anspruch 1, wobei die erste Probe vor der Anwendung einer neoadjuvanten Therapie von der Person gewonnen wird und die zweite Probe nach der Anwendung der neoadjuvanten Therapie und vor der Anwendung einer anderen Therapie von der Person gewonnen wird; oder wobei die erste Probe vor der Anwendung eines Therapiezyklus von der Person gewonnen wird und die zweite Probe nach der Anwendung des Therapiezyklus und vor der Anwendung eines nächsten Therapiezyklus von der Person gewonnen wird.

3. Verfahren nach Anspruch 1, wobei die Schritte (a) bis (f) mehrere Therapiezyklen hindurch wiederholt werden.

4. Verfahren nach Anspruch 3, wobei eine Zunahme der Menge von kurzen RE-Zielen in der zweiten Probe im Vergleich zu der ersten Probe oberhalb eines Schwellenwertes die Therapie als wirkungslos identifiziert.

5. Verfahren nach Anspruch 1, wobei eine Zunahme der Menge von kurzen RE-Zielen in der zweiten Probe im Vergleich zu der ersten Probe oberhalb eines Schwellenwertes den Patienten als einen Patienten mit einer fortschreitenden Erkrankung identifiziert oder wobei eine Zunahme der Menge von kurzen RE-Zielen in der zweiten Probe im Vergleich zu der ersten Probe oberhalb eines Schwellenwertes den Patienten als einen Patienten mit einer minimalen Resterkrankung, *minimum residual disease* - (MRD), identifiziert.

6. Verfahren nach Anspruch 1, wobei die kurzen RE-Ziele eine Länge von 70 bp bis ungefähr 130 bp oder von 60 bp bis 120 bp aufweisen; vorzugsweise das erste kurze RE-Ziel eine Länge zwischen 70 und 80 bp aufweist und das zweite kurze RE-Ziel eine Länge zwischen 105 und 120 bp aufweist.

7. Verfahren nach Anspruch 1, wobei das qPCR-Verfahren Primerpaare verwendet, die in Tabelle 2A oder 2B ausgewiesen sind.

8. Verfahren nach Anspruch 1, wobei das Paar aus Vorwärtsprimer und Rückwärtsprimer zur Amplifizierung der kurzen Zielsequenzen aus den folgenden Vorwärts- und Rückwärtsprimerpaaren ausgewählt wird:
| Fragmentname | Größe | Primertyp | Primersequenz | SEQ ID NR. |
|---|---|---|---|---|
| Yb8-80bp | 80 bp | Vorwärts | GGAAGCGGAGCTTGCAGTGA | |
| | | Rückwärts | AGACGGAGTCTCGCTCTGTCGC | |
| Yb8-71bp | 71 bp | Vorwärts | CTTGCAGTGAGCCGAGATT | |
| | | Rückwärts | GAGACGGAGTCTCGCTCTGTC | |
| Yb8-97bp | 97 bp | Vorwärts | GTGGCTCACGCCTGTAAT | |
| | | Rückwärts | GTGGCTCACGCCTGTAAT | |
| Yb8-105bp | 105 bp | Vorwärts | AGGCAGGAGAATGGCGTGAACC | |
| | | Rückwärts | AGACGGAGTCTCGCTCTGTCGC | |
| Yb8-120bp | 120 bp | Vorwärts | TGGATCATGAGGTCAGGAGAT | |
| | | Rückwärts | CCGAGTAGCTGGGACTACA | |
| SVA-100bp | 100 bp | Vorwärts | AATGGCGGCTTTGTGGAATA | |
| | | Rückwärts | GTCTCCCATGTCTACTTCTTTCTAC | |
| SVA-101bp | 101 bp | Vorwärts | AACCCTGTGCTCTCTGAAAC | |
| | | Rückwärts | ACGCTGCCTTCAAGCAT | |
| SVA-103bp | 103 bp | Vorwärts | GCCCAACAGCTCATTGAGAA | |
| | | Rückwärts | ACGGCAACCATCCGATTT | |
| SVA-104bp | 104 bp | Vorwärts | TGTCCACTCAGGGTTAAATGG | |
| | | Rückwärts | GATTAGGGATTGGTGATAACTCTTA | |
| SVA-106bp | 106 bp | Vorwärts | TGTGTCCACTCAGGGTTAAAT | |
| | | Rückwärts | GATTAGGGATTGGTGATGACTCT | |
| SVA-106bp-v2 | 106 bp | Vorwärts | TGTGCCCAACAGCTCATT | |
| | | Rückwärts | ACGGCAACCATCCGATTT | |
| SVA-116bp | 116 bp | Vorwärts | CTGTGTCCACTCAGGGTTAAATG | |
| | | Rückwärts | ATTACTTGAGATTAGGGATTGGTGATG | |
| SVA-116bp-v2 | 116 bp | Vorwärts | CCCAACAGCTCATTGAGAACG | |
| | | Rückwärts | CTTTCTACACAGACACGGCAA | |
| SVA-118bp | 118 bp | Vorwärts | CTCTCTGAAACATGTGCTGTGT | |
| | | Rückwärts | GGGATTGGTGATGACTCTTAACG | |
| SVA-118bp-v2 | 118 bp | Vorwärts | CTGTGTCCACTCAGGGTTAAAT | |
| | | Rückwärts | TGATTACTTGAGATTAGGGATTGGT | |
| SVA-126bp | 126 bp | Vorwärts | CTGTGTCCACTCAGGGTTAAAT | |
| | | Rückwärts | TGTGTCCCTGATTACTTGAGATTAG | |
| SVA-126bp-V2 | 126 bp | Vorwärts | CCTGTTGATCTGTGACCTTACC | |
| | | Rückwärts | ACGCTGCCTTCAAGCAT | |
| SVA-128bp | 128 bp | Vorwärts | GTTGCCGTGTCTGTGTAGAA | |
| | | Rückwärts | TTTCAGAGAGCACAGGGTTG | |
| SVA-132bp | 132 bp | Vorwärts | AACCCTGTGCTCTCTGAAAC | |
| | | Rückwärts | GATTAGGGATTGGTGATAACTCTTA | |

9. Verfahren nach Anspruch 1, ferner umfassend einen Schritt des Hinzufügens einer synthetischen DNA-Sequenz als interne Positivkontrolle vor Schritt (b), das Quantifizieren der internen Positivkontrolle in Schritt (b) und das Verwenden des quantitativen Ergebnisses der internen Positivkontrolle in dem Vergleichsschritt, um die Genauigkeit und Zuverlässigkeit des Vergleichsschrittes zu verbessern; wobei vorzugsweise die Verwendung der internen Positivkontrolle eine Bestimmung einer Konzentration zellfreier DNA in der Probe ermöglicht.

10. Verfahren nach Anspruch 1, wobei die Schritte (b)-(f) in einem einzigen Röhrchen oder Well durchgeführt werden.

11. Verfahren nach Anspruch 1, wobei der Bereitstellungsschritt ferner das Bereitstellen einer Hybridisierungssonde umfasst, die an das RE-Ziel hybridisiert; wobei vorzugsweise die Hybridisierungssonde eine beobachtbare Markierung aufweist; wobei besonders bevorzugt die beobachtbare Markierung eine Fluoreszenzmarkierung ist.

12. Verfahren nach Anspruch 1, wobei die Menge des ersten und des zweiten kurzen RE-Ziels in der ersten und der zweiten Probe, amplifiziert im Zuge des Verfahrens der quantitativen Polymerase-Kettenreaktion, *quantitative polymerase chain reaction* - (qPCR), unter Verwendung eines elektrischen Biosensors gemessen wird.

13. Verfahren nach Anspruch 1, wobei die quantifizierte Menge von kurzen RE-Zielen eine Krebszelle bei einer Gesamtzahl von 500.000 Zellen oder mehr, bei einer Gesamtzahl von 1.000.000 Zellen oder mehr oder bei 1.500.000 Zellen oder mehr bei dem Patienten darstellt.

14. Multiplexsystem zur Durchführung eines Verfahrens nach einem der Ansprüche 1 bis 13 zur Identifizierung einer wirkungslosen neoadjuvanten oder Krebsbehandlung oder zur Kennzeichnung von Krebs oder MRD beim Menschen, wobei das System Folgendes umfasst:
a. eine Probe von Serum, Plasma, Urin oder einer anderen biologischen Flüssigkeit von einem Menschen, wobei die Probe zellfreie DNA umfasst, die zellfreie DNA zwei kurze RE-Ziele umfasst, die kurzen RE-Ziele eine Länge zwischen 60 bp und 135 bp aufweisen, mit der Maßgabe, dass die zwei kurzen RE-Ziele sich in ausreichendem Maße in ihrer Größe unterscheiden, um ihre Amplicone zu unterscheiden, wobei die Probe ferner eine interne Positivkontrolle, *internal positive control -* (IPC), umfasst, die synthetische DNA umfasst;
b. eine TaqMan^{®}-Sonde, die sowohl den kurzen RE-Zielen als auch der IPC entspricht, wobei jede Sonde eine detektierbare Markierung umfasst, die sich von den Markierungen, die in die anderen Sonden eingebracht sind, unterscheidet;
c. einen Vorwärtsprimer und einen Rückwärtsprimer zur Amplifizierung sowohl des kurzen RE-Ziels als auch der IPC;
d. einen DNA-Standard zur Erzeugung von Standardkurven für RE-Ziele;
e. ein qPCR-System zur gleichzeitigen Amplifizierung der kurzen RE-Ziele und der IPC und zur Erzeugung einer Schwellenzyklusanzahl für jedes Ziel; und
f. ein qPCR-Datenanalysesystem zur Erzeugung von DNA-Quantifizierungswerten für jedes RE-Ziel durch Interpolation unter Verwendung von Schwellenzyklusanzahlen und Standardkurven und zur Verwendung der DNA-Quantifizierungswerte zur Erzeugung einer Angabe bezüglich der Integrität der zellfreien DNA.

15. System nach Anspruch 14, wobei die zwei kurzen RE-Ziele sich in ihrer Länge um mindestens 10 bp, 15 bp, 20 bp oder 25 bp unterscheiden.

## Revendications

1. Procédé pour quantifier l'intégrité de l'ADN humain acellulaire circulant et mettre en oeuvre un traitement d'un patient, ledit procédé comprenant :
(a) la fourniture d'un premier et un deuxième échantillon de sérum, de plasma, d'urine ou d'un autre fluide biologique d'un sujet, le premier et le deuxième échantillon étant obtenus à au moins une semaine d'intervalle, à au moins deux semaines d'intervalle, à au moins trois semaines d'intervalle ou à au moins quatre semaines d'intervalle, les premier et deuxième échantillons comprenant de l'ADN humain acellulaire (ADNcf), l'ADNcf comprenant une première et une deuxième séquence cible courte d'éléments intercalés rétrotransposables (RE) ayant une longueur comprise entre environ 60 paires de bases et environ 135 paires de bases, la première et la deuxième cible courte différant en longueur ;
(b) la quantification de chacune des première et deuxième cibles RE courtes dans les premier et deuxième échantillons à l'aide d'un procédé de réaction en chaîne par polymérase quantitative (qPCR) ;
(c) l'obtention pour chacune des cibles RE quantifiées dans les premier et deuxième échantillons d'un nombre de cycles seuil ;
(d) la comparaison du nombre de cycles seuil de chaque cible RE quantifiée à une courbe standard pour déterminer une quantité de chacune des cibles RE quantifiées qui étaient présentes dans les échantillons, la quantité de cibles RE courtes dans les deuxièmes échantillons étant indicative de l'intégrité de l'ADN acellulaire circulant ;
(e) la détermination d'une augmentation de la quantité de cibles RE courtes dans le deuxième échantillon par rapport au premier échantillon au-delà d'un niveau de seuil, et
(f) la détermination d'un traitement pour un patient présentant une augmentation de la quantité de cibles RE courtes dans le deuxième échantillon en comparaison du premier échantillon au-delà d'un niveau de seuil.

2. Procédé selon la revendication 1, dans lequel le premier échantillon est obtenu du sujet avant l'administration d'un néoadjuvant et le deuxième échantillon est obtenu du sujet après l'administration du néoadjuvant et avant l'administration d'une autre thérapie ; ou dans lequel le premier échantillon est obtenu du sujet avant l'administration d'un cycle de thérapie et le deuxième échantillon est obtenu du sujet après l'administration du cycle de thérapie et avant l'administration d'un prochain cycle de thérapie.

3. Procédé selon la revendication 1, dans lequel les étapes (a) à (f) sont répétées à travers plusieurs cycles de thérapie.

4. Procédé selon la revendication 3, dans lequel une augmentation de la quantité de cibles RE courtes dans le deuxième échantillon en comparaison du premier échantillon au-dessus d'un niveau de seuil identifie la thérapie comme inefficace.

5. Procédé selon la revendication 1, dans lequel une augmentation de la quantité de cibles RE courtes dans le deuxième échantillon en comparaison du premier échantillon au-delà d'un niveau de seuil identifie le patient comme présentant une maladie progressive, ou dans laquelle une augmentation de la quantité de cibles RE courtes dans le deuxième échantillon en comparaison du premier échantillon au-delà d'un niveau de seuil identifie le patient comme présentant une maladie résiduelle minime (MRM).

6. Procédé selon la revendication 1, dans lequel les cibles RE courtes ont une longueur de 70 pb à environ 130 pb, ou de 60 pb à 120 pb ; de préférence, la première cible RE courte a une longueur comprise entre 70 et 80 pb, et la deuxième cible RE courte a une longueur comprise entre 105 et 120 pb.

7. Procédé selon la revendication 1, dans lequel le procédé qPCR utilise des paires d'amorces présentées dans le tableau 2A ou 2B.

8. Procédé selon la revendication 1, dans lequel la paire consistant en une amorce avant et une amorce arrière pour amplifier les séquences cibles courtes est choisie parmi les paires d'amorce avant et d'amorce arrière suivantes :
| Nom du fragment | Taille | Type d'amorce | Séquence d'amorce | SEQ ID NO |
|---|---|---|---|---|
| Yb8-80bp | 80 bp | Avant | GGAAGCGGAGCTTGCAGTGA | |
| | | Arrière | AGACGGAGTCTCGCTCTGTCGC | |
| Yb8-71bp | 71 bp | Avant | CTTGCAGTGAGCCGAGATT | |
| | | Arrière | GAGACGGAGTCTCGCTCTGTC | |
| Yb8-97bp | 97 bp | Avant | GTGGCTCACGCCTGTAAT | |
| | | Arrière | GTGGCTCACGCCTGTAAT | |
| Yb8-105bp | 105 bp | Avant | AGGCAGGAGAATGGCGTGAACC | |
| | | Arrière | AGACGGAGTCTCGCTCTGTCGC | |
| Yb8-120bp | 120 bp | Avant | TGGATCATGAGGTCAGGAGAT | |
| | | Arrière | CCGAGTAGCTGGGACTACA | |
| SVA-100bp | 100 bp | Avant | AATGGCGGCTTTGTGGAATA | |
| | | Arrière | GTCTCCCATGTCTACTTCTTTCTAC | |
| SVA-101bp | 101 bp | Avant | AACCCTGTGCTCTCTGAAAC | |
| | | Arrière | ACGCTGCCTTCAAGCAT | |
| SVA-103bp | 103 bp | Avant | GCCCAACAGCTCATTGAGAA | |
| | | Arrière | ACGGCAACCATCCGATTT | |
| SVA-104bp | 104 bp | Avant | TGTCCACTCAGGGTTAAATGG | |
| | | Arrière | GATTAGGGATTGGTGATAACTCTT | |
| SVA-106bp | 106 bp | Avant | TGTGTCCACTCAGGGTTAAAT | |
| | | Arrière | GATTAGGGATTGGTGATGACTCT | |
| SVA-106bp-v2 | 106 bp | Avant | TGTGCCCAACAGCTCATT | |
| | | Arrière | ACGGCAACCATCCGATTT | |
| SVA-116bp | 116 bp | Avant | CTGTGTCCACTCAGGGTTAAATG | |
| | | Arrière | ATTACTTGAGATTAGGGATTGGTG | |
| SVA-116bp-v2 | 116 bp | Avant | CCCAACAGCTCATTGAGAACG | |
| | | Arrière | CTTTCTACACAGACACGGCAA | |
| SVA-118bp | 118 bp | Avant | CTCTCTGAAACATGTGCTGTGT | |
| | | Arrière | GGGATTGGTGATGACTCTTAACG | |
| SVA-118bp-v2 | 118 bp | Avant | CTGTGTCCACTCAGGGTTAAAT | |
| | | Arrière | TGATTACTTGAGATTAGGGATTGG | |
| SVA-126bp | 126 bp | Avant | CTGTGTCCACTCAGGGTTAAAT | |
| | | Arrière | TGTGTCCCTGATTACTTGAGATTA | |
| SVA-126bp-V2 | 126 bp | Avant | CCTGTTGATCTGTGACCTTACC | |
| | | Arrière | ACGCTGCCTTCAAGCAT | |
| SVA-128bp | 128 bp | Avant | GTTGCCGTGTCTGTGTAGAA | |
| | | Arrière | TTTCAGAGAGCACAGGGTTG | |
| SVA-132bp | 132 bp | Avant | AACCCTGTGCTCTCTGAAAC | |
| | | Arrière | GATTAGGGATTGGTGATAACTCTT | |

9. Procédé selon la revendication 1, comprenant en outre une étape d'ajout d'une séquence d'ADN synthétique en tant que contrôle positif interne avant l'étape (b), de quantification du contrôle positif interne à l'étape (b), et d'utilisation du résultat quantitatif du contrôle positif interne dans l'étape de comparaison pour améliorer la précision et la fiabilité de l'étape de comparaison ; de préférence, l'utilisation du contrôle positif interne permettant de déterminer une concentration d'ADN acellulaire dans l'échantillon.

10. Procédé selon la revendication 1, les étapes (b)-(f) étant effectuées dans un seul tube ou puits.

11. Procédé selon la revendication 1, l'étape de fourniture comprenant en outre la fourniture d'une sonde d'hybridation qui s'hybride à la cible RE ; de préférence la sonde d'hybridation incluant une étiquette observable ; plus préférentiellement l'étiquette observable étant une étiquette fluorescente.

12. Procédé selon la revendication 1, dans lequel la quantité des première et deuxième cibles RE courtes dans les premier et deuxième échantillons amplifiés dans le procédé de réaction en chaîne par polymérase quantitative (qPCR) sont mesurées à l'aide d'un biocapteur électrique.

13. Procédé selon la revendication 1, dans lequel la quantité de cible RE courte quantifiée représente une cellule cancéreuse sur 500 000 cellules totales ou plus, 1 000 000 cellules totales ou plus ou 1 500 000 cellules ou plus chez le patient.

14. Système multiplexé pour la mise en oeuvre d'un procédé selon l'une quelconque des revendications 1 à 13 pour l'identification d'un traitement néoadjuvant ou anticancéreux inefficace, ou pour la caractérisation du cancer ou de la MRM chez l'homme, le système comprenant :
a. un échantillon de sérum, de plasma, d'urine ou d'un autre liquide biologique d'un être humain, l'échantillon comprenant de l'ADN acellulaire, l'ADN acellulaire comprenant deux cibles RE courtes, les cibles RE courtes ayant une longueur comprise entre 60 pb et 135 pb, à condition que les deux cibles RE courtes diffèrent en taille suffisamment pour distinguer leurs amplicons, l'échantillon comprenant en outre un contrôle positif interne (CPI) comprenant de l'ADN synthétique ;
b. une sonde TaqMan^{®} correspondant à chacune des cibles RE courtes et à l'IPC, chaque sonde comprenant une étiquette détectable distincte des étiquettes incorporées dans les autres sondes ;
c. une amorce avant et une amorce arrière pour l'amplification de chacune des cibles RE courtes et de l'IPC ;
d. un standard d'ADN pour générer des courbes standard pour les cibles RE ;
e. un système qPCR pour l'amplification simultanée des cibles RE courtes et de l'IPC et pour la production d'un nombre de cycles seuil pour chaque cible ; et
f. un système d'analyse des données qPCR pour produire des valeurs de quantification de l'ADN pour chaque cible RE par interpolation à l'aide des nombres de cycles seuils et des courbes standard, et pour utiliser les valeurs de quantification de l'ADN pour produire une indication de l'intégrité de l'ADN acellulaire.

15. Système selon la revendication 14, dans laquelle les deux cibles RE courtes diffèrent en longueur d'au moins 10 pb, 15 pb, 20 pb ou 25 pb.
